(19) **Europäisches Patentamt**
**European Patent Office**
**Office européen des brevets**

(11) **EP 3 847 961 B1**

(12) # EUROPEAN PATENT SPECIFICATION

(45) Date of publication and mention
of the grant of the patent:
**19.04.2023 Bulletin 2023/16**

(21) Application number: **18932484.1**

(22) Date of filing: **03.09.2018**

(51) International Patent Classification (IPC):
***A61B 5/11*** *(2006.01)*

(52) Cooperative Patent Classification (CPC):
**A61B 5/112; A61B 5/1123; A61B 5/6802;
A61B 5/6829; A61B 5/7246;** A61B 2562/0219

(86) International application number:
**PCT/JP2018/032669**

(87) International publication number:
**WO 2020/049621 (12.03.2020 Gazette 2020/11)**

(54) **WALKING STATE DETERMINATION PROGRAM, WALKING STATE DETERMINATION METHOD, AND INFORMATION PROCESSING DEVICE**

PROGRAMM ZUR BESTIMMUNG DES GEHZUSTANDS, VERFAHREN ZUR BESTIMMUNG DES GEHZUSTANDS UND VORRICHTUNG ZUR INFORMATIONSVERARBEITUNG

PROGRAMME DE DÉTERMINATION DE STATUT DE DÉAMBULATION, PROCÉDÉ DE DÉTERMINATION STATUT DE DÉAMBULATION, ET DISPOSITIF DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.07.2021 Bulletin 2021/28**

(73) Proprietor: **FUJITSU LIMITED
Kanagawa 211-8588 (JP)**

(72) Inventors:
• **HOTTA, Shinji
Kawasaki-shi, Kanagawa 211-8588 (JP)**
• **KOMABA, Yusuke
Kawasaki-shi, Kanagawa 211-8588 (JP)**
• **YAGINUMA, Yoshinori
Kawasaki-shi, Kanagawa 211-8588 (JP)**

(74) Representative: **Hoffmann Eitle
Patent- und Rechtsanwälte PartmbB
Arabellastraße 30
81925 München (DE)**

(56) References cited:
WO-A1-2017/199305    JP-A- 2010 172 758
US-A1- 2004 064 286    US-A1- 2011 246 123
US-A1- 2011 246 123    US-A1- 2012 136 573

• ZHANG ZELUN ET AL: "Improved Use of Foot Force Sensors and Mobile Phone GPS for Mobility Activity Recognition", IEEE SENSORS JOURNAL, IEEE SERVICE CENTER, NEW YORK, NY, US, vol. 14, no. 12, 1 December 2014 (2014-12-01), pages 4340-4347, XP011562444, ISSN: 1530-437X, DOI: 10.1109/JSEN.2014.2331463 [retrieved on 2014-10-22]

EP 3 847 961 B1

Processed by Luminess, 75001 PARIS (FR)

**Description**

TECHNICAL FIELD

The present invention relates to a walking state determination program, a walking state determination method, and an information processing apparatus.

BACKGROUND ART

In recent years, the number of elderly people has been increased, and it has been difficult for doctors to examine and treat each elderly person. If information from which a health condition of a person can be grasped is automatically measured and the health condition can be grasped from a remote place, the doctors can treat the person to be treated. For example, a walking feature such as a walking speed or a stride length is an important indicator to grasp the health condition of the person. Therefore, a technique has been desired for quantifying a walking feature of a person from information of a sensor attached to a foot or the like.

As related art, there is a technique for setting a part where peaks detected by a predetermined system frequently occur in inertial data of both feet as a walking section and extracting a walking speed, a stride length, or the like using data in the walking section (for example, refer to Non-patent Document 1).

Furthermore, there is a technique for calculating at least one of an early leg swinging time, a late leg swinging time, and an entire leg swinging time on the basis of a detection signal that changes according to a walking movement and determining a type of the walking movement on the basis of at least one of the early leg swinging time, the late leg swinging time, and the entire leg swinging time (for example, refer to Patent Document 1). Furthermore, there is a technique for estimating a walking ability of a user from a temporal change in each acceleration detected by an accelerometer attached to a waist of the user and a relationship between a stored estimated index and the walking ability (for example, refer to Patent Document 2). Furthermore, there is a technique for calculating an absolute value of each acceleration vector, an average value of the absolute values, a standard deviation, and a periodicity regarding acceleration vector data that is continuously collected and determining that the person is in a walking state in a case where the standard deviation is within a predetermined range and the periodicity is larger than a predetermined value (for example, refer to Patent Document 3). Furthermore, there is a technique for identifying a posture of a subject using acceleration data from a sensor device attached to the subject, calculating a behavior feature amount used to identify a behavior that may be taken with the posture, and identifying one behavior from among the plurality of behaviors that may be made with the identified posture on the basis of the behavior feature amount (for example, refer to Patent Document 4). Furthermore, there is a technique for synthesizing measured values of axes measured by an acceleration sensor to calculate a synthesized value, detecting a fluctuation in a synthesized value that satisfies a predetermined condition, and not counting the fluctuation in the synthesized value that satisfies the predetermined condition that occurs within a predetermined period as the number of steps after detecting the fluctuation in the synthesized value that satisfies the predetermined condition when calculating the number of steps from the number of times of detection (for example, refer to Patent Document 5).

CITATION LIST

PATENT DOCUMENT

Patent Document 1: Japanese Laid-open Patent Publication No. 2015-136582
Patent Document 2: Japanese Laid-open Patent Publication No. 2007-125368
Patent Document 3: Japanese Laid-open Patent Publication No. 2010-172758
Patent Document 4: Japanese Laid-open Patent Publication No. 2009-39466
Patent Document 5: Japanese Laid-open Patent Publication No. 2017-59089

US 2011/246 123 A1 describes a method for monitoring kinetic motion including: capturing acceleration data of a human body of interest from a plurality of points on the human body of interest; using the plurality of points that correlate to parts of the human body of interest to determine a position or view of the human body of interest, wherein the position or view includes a kinetic signature comprising motion characteristics; and displaying a live representation of the human body of interest by using the determined position or view of the human body of interest.

NON-PATENT DOCUMENT

Non-Patent Document 1: Fraccalo, "Real-world Gyroscope-based Gait Event Detection and Gait Feature Extraction", 2014

## SUMMARY OF INVENTION

### TECHNICAL PROBLEM

However, according to the related art, it is difficult to accurately determine whether or not a person is walking from the information of the sensor attached to the foot or the like. There is a case where a movement for "carrying", "lifting", "putting", "shaking" or the like the sensor before an object attaches the sensor to the foot, and such a movement has a high degree of freedom, and there is a possibility that various patterns of data is generated. Therefore, for example, when the sensor is attached to the foot, a peak may be detected and it may be erroneously determined as a walking section.

In one aspect, an object of the present invention is to improve accuracy of determination regarding whether or not a person is in a walking state.

### SOLUTION TO PROBLEM

The above problem is solved by the subject matter of the independent claims. Examples and technical descriptions of apparatuses, products and/or methods in the description and/or drawings which are not covered by the claims are presented not as embodiments of the invention but as background art or examples useful for understanding the invention. According to one such example, a walking state determination program is provided that includes acquiring first measurement data and second measurement data that respectively indicate transitions of measured values measured by a first sensor and a second sensor respectively attached to a left and a right feet in a predetermined period, calculating a correlation degree indicating a degree of a correlation between the transition of the measured value indicating by the acquired first measurement data and the transition of the measured value indicated by the acquired second measurement data, and determining whether or not walking is performed on the basis of the calculated correlation degree.

### ADVANTAGEOUS EFFECTS OF INVENTION

According to one aspect of the present invention, an effect is obtained that it is possible to improve accuracy of determination regarding whether or not a person is in a walking state.

### BRIEF DESCRIPTION OF DRAWINGS

FIG. 1 is an explanatory diagram illustrating an example of a walking state determination method according to an embodiment.
FIG. 2 is an explanatory diagram illustrating an exemplary system configuration of an information processing system 200.
FIG. 3 is an explanatory view illustrating an example in which sensors are attached.
FIGs. 4A to 4D are an explanatory view illustrating an axial direction of inertial data.
FIG. 5 is a block diagram illustrating an exemplary hardware configuration of an information processing apparatus 101.
FIG. 6 is an explanatory diagram illustrating an example of storage content of an inertial data DB 220.
FIG. 7 is a block diagram illustrating an exemplary functional configuration of the information processing apparatus 101.
FIG. 8 is an explanatory diagram illustrating a specific example of provisional walking section information.
FIG. 9 is an explanatory diagram illustrating a specific example of measurement data at the time of non-walking.
FIG. 10 is an explanatory diagram illustrating a determination example of a walking period.
FIG. 11 is an explanatory diagram illustrating a setting example of a window width.
FIG. 12 is an explanatory diagram illustrating a specific example of measurement data at the time of walking with legs flexed 90 degrees.
FIG. 13 is an explanatory diagram illustrating a comparative example of peaks of gyro data.
FIG. 14A is an explanatory diagram (part 1) illustrating a screen example of a monitoring screen.
FIG. 14B is an explanatory diagram (part 2) illustrating a screen example of the monitoring screen.

FIG. 15 is a flowchart illustrating an example of a walking state determination processing procedure of the information processing apparatus 101.

FIG. 16 is a flowchart illustrating an example of a specific processing procedure of second correlation degree calculation processing.

FIG. 17 is an explanatory diagram illustrating an example of threshold determination.

DESCRIPTION OF EMBODIMENTS

Embodiments of a walking state determination program, a walking state determination method, and an information processing apparatus according to the present invention will be described below in detail with reference to the drawings.

(Embodiment)

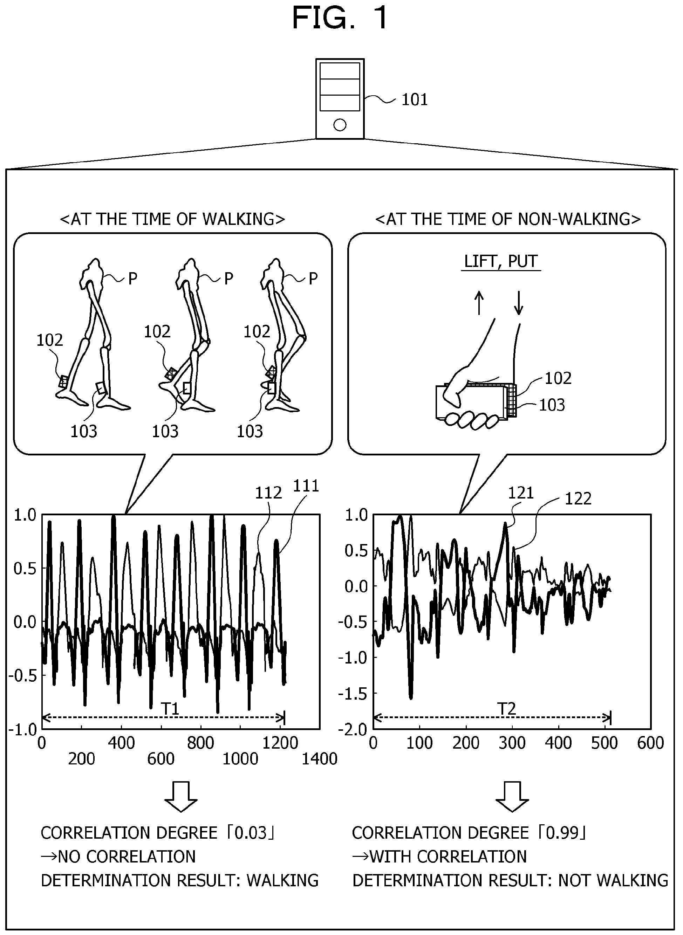

FIG. 1 is an explanatory diagram illustrating an example of a walking state determination method according to an embodiment. In FIG. 1, an information processing apparatus 101 is a computer that determines a walking period. The walking period is a period in which a measurement target has walked. The measurement target is a target of which a value regarding a movement is measured and may be, for example, a human or an animal other than a human. More specifically, for example, the measurement target is a patient who undergoes diagnosis, treatment, follow-up, health management, or the like by a doctor.

Here, as a technique for extracting a walking feature from information of a sensor attached to a foot or the like, there is a technique for setting a portion where peaks detected by a predetermined system frequently occur in inertial data of both feet as a walking section and extracting a walking speed, a stride length, or the like using data in the walking section. The inertial data is, for example, information regarding an angular velocity measured by a sensor attached to each of right and left feet.

However, depending on an illness or a recovery state from an illness, a walking style of an object may differ from that when the object is healthy. Therefore, in a case where the portion in which the peaks frequently occur is determined as a walking section, if the peaks are different from those when the object is healthy, there is a possibility that detection of a walking section may be omitted.

Furthermore, before the object attaches the sensor on the foot, movements such as "carrying", "lifting", "putting", "shaking", or the like may occur. Such a movement has a high degree of freedom, and there is a possibility that various patterns of data is generated. Therefore, for example, when the sensor is attached to the foot, a peak may be detected and it may be erroneously determined as a walking section.

Note that it is considered that the object or a third party gives a trigger for starting or ending data acquisition and only data in a period when the object walks is acquired. For example, it is considered that data is sent from the sensor attached on the feet to a smartphone through wireless communication, a data acquisition start trigger is given through an application of the smartphone after the sensor is attached, and a data acquisition end trigger is given before the sensor is removed. However, it takes time and effort to manually give the data acquisition start/end trigger, and there is a possibility for forgetting to give the trigger. If the target or the third party forgets to give the trigger, it is not possible to guarantee a determination result.

Therefore, in the present embodiment, a walking state determination method will be described for improving accuracy of determination regarding whether or not a person is in a walking state using tendency such that inertial data of a left foot and inertial data of a right foot have no correlation at the time of walking. Hereinafter, an example of walking state determination processing of the information processing apparatus 101 will be described.

Here, a case is assumed where a first sensor 102 and a second sensor 103 are respectively attached to the left and right feet of an object P. The foot is a part that supports the body and is used to walk. For example, the first sensor 102 and the second sensor 103 are respectively attached around ankles of the left and right feet.

(1) The information processing apparatus 101 acquires first measurement data and second measurement data that respectively indicate transitions of measured values measured by the first sensor 102 and the second sensor 103 in a predetermined period T. Here, the predetermined period T is a target period in which it is determined whether or not the predetermined period T is a walking period.

Note that the predetermined period T may be a period that is estimated as the walking period according to an existing technique different from this walking state determination method. In this case, it can be determined that the walking period estimated by the existing technique is correct by this walking state determination method.

In the example in FIG. 1, a case is assumed where first measurement data 111 and second measurement data 112 are acquired for a predetermined period T1 and first measurement data 121 and second measurement data 122 are acquired for a predetermined period T2.

(2) The information processing apparatus 101 calculates a correlation degree between the first measurement data and the second measurement data that have been acquired. Here, the correlation degree is a value that indicates a degree of a correlation between the transition of the measured value indicated by the first measurement data and the transition of the measured value indicated by the second measurement data. For example, the correlation degree is an absolute value of a correlation coefficient indicated by a numerical value within a range "equal to or more than zero and equal to or less than one".

In the example in FIG. 1, a case is assumed where "0.03" is calculated as a correlation degree between the first measurement data 111 and the second measurement data 112 and "0.99" is calculated as a correlation degree between the first measurement data 121 and the second measurement data 122.

(3) The information processing apparatus 101 determines whether or not walking is performed on the basis of the calculated correlation degree. Here, at the time walking, when one foot is swinging, another foot (part lower than knee) is stopped. The swing is a swing of the foot from a time when the toe leaves from the ground to a time the heel is grounded after swinging the foot forward.

Therefore, at the time of walking, the measurement data of the left foot and the measurement data of the right foot tend to have no correlation. In other words, in a case where there is a correlation between the measurement data of the left foot and the measurement data of the right foot, it can be determined that this is a period of a movement other than walking, that is, a non-walking period.

For example, the movements such as "carrying", "lifting", "putting", or "shaking" the sensor other than walking may be performed on a plurality of sensors (for example, first sensor 102, second sensor 103) at the same time, not one sensor. In this case, the correlation between the measurement data of the left foot and the measurement data of the right foot increases, and it can be assumed that this period be a non-walking period.

Therefore, the information processing apparatus 101 determines that the movement is walking, for example, in a case where the calculated correlation degree is less than a first threshold. On the other hand, in a case where the correlation degree is equal to or more than the first threshold, the information processing apparatus 101 determines that the movement is not walking. The first threshold can be set to any value and, for example, set to a value of about 0.2.

In the example in FIG. 1, the first threshold is set to "0.2". In this case, the correlation degree "0.03" between the first measurement data 111 and the second measurement data 112 is less than the first threshold. Therefore, the information processing apparatus 101 determines that the movement is walking. On the other hand, the correlation degree "0.99" between the first measurement data 121 and the second measurement data 122 is equal to or more than the first threshold. Therefore, the information processing apparatus 101 determines that the movement is not walking.

In this way, according to the information processing apparatus 101, it is possible to calculate the correlation degree between the transition of the measured value indicated by the first measurement data and the transition of the measured value indicated by the second measurement data and to determine whether or not the movement is walking on the basis of the calculated correlation degree. As a result, at the time of walking, it is possible to improve the accuracy of the determination regarding whether or not the object P is in a walking state by using the tendency such that the measurement data of the left foot and the measurement data of the right foot have no correlation.

(Exemplary System Configuration of Information Processing System 200)

Next, an exemplary system configuration of an information processing system 200 including the information processing apparatus 101 illustrated in FIG. 1 will be described. The information processing system 200 is applied to, for example, a remote monitoring system that monitors a health condition of the object P.

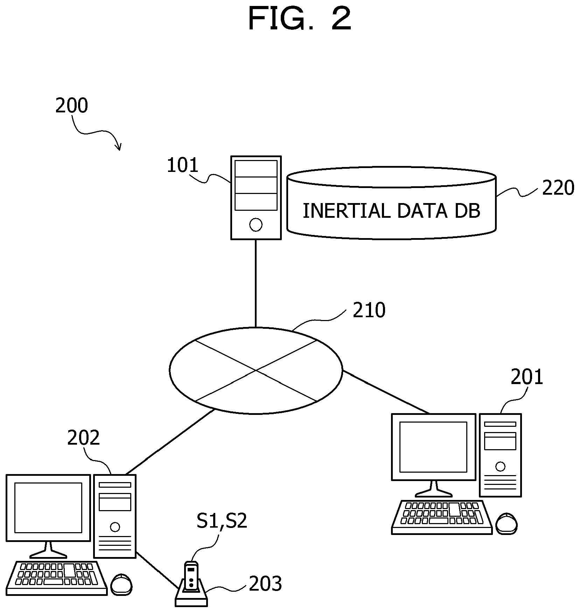

FIG. 2 is an explanatory diagram illustrating an exemplary system configuration of the information processing system 200. In FIG. 2, the information processing system 200 includes the information processing apparatus 101, an administrator terminal 201, and an object terminal 202. In the information processing system 200, the information processing apparatus 101, the administrator terminal 201, and the object terminal 202 are connected via a wired or wireless network 210. Examples of the network 210 include a local area network (LAN), a wide area network (WAN), the Internet, or the like. Note that, as another system configuration example, the information processing apparatus 101 and the object terminal 202 may be implemented as a single device, and the entire device may be integrally implemented as a stand-alone system.

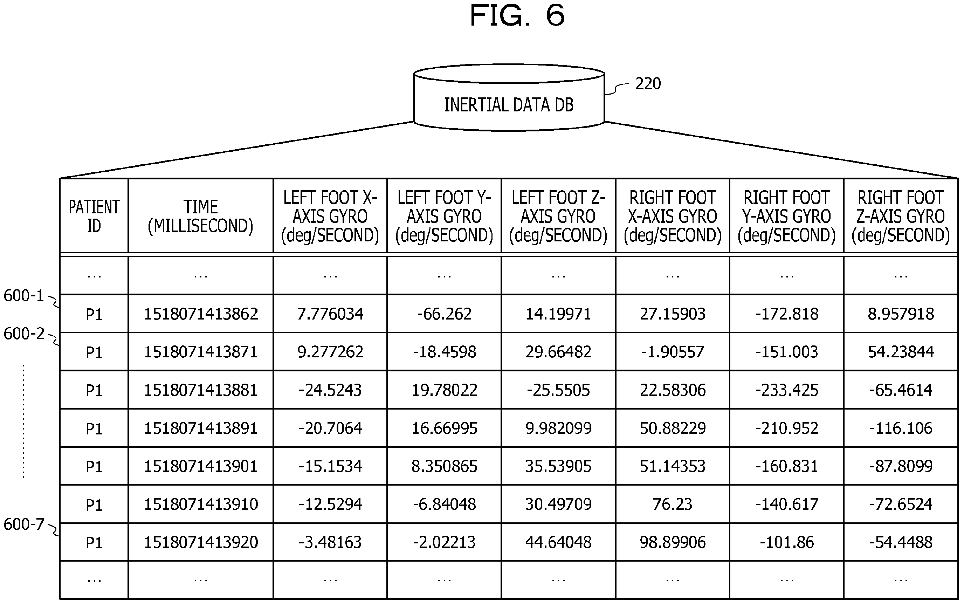

Here, the information processing apparatus 101 includes an inertial data database (DB) 220 and determines a walking section of the object P. The walking section is a walking period and is, for example, a period in which the object P walks in a state where sensors S1 and S2 are attached on feet. The information processing apparatus 101 is, for example, a server. Note that storage content of the inertial data DB 220 will be described later with reference to FIG. 6.

The administrator terminal 201 is a computer used by an administrator. The administrator is, for example, a medical worker such as a doctor or a nurse in a medical institution. The administrator terminal 201 is, for example, a personal computer (PC), a tablet PC, a smartphone, or the like.

The object terminal 202 is a computer used by the object P. The object P is, for example, a patient who undergoes diagnosis, treatment, follow-up, and the like by a medical worker. The object terminal 202 includes a reading unit 203 and can acquire data from the sensors S1 and S2. The object terminal 202 is, for example, a PC, a tablet PC, a smartphone, or the like.

The sensors S1 and S2 are sensors that measure values regarding movements, and are respectively attached to the left and right feet of the object P. For example, each of the sensors S1 and S2 is a gyro sensor that measures angular velocities in a plurality of directions. The first sensor 102 illustrated in FIG. 1 corresponds to, for example, the sensor S1. Furthermore, the second sensor 103 illustrated in FIG. 1 corresponds to, for example, the sensor S2.

Each of the sensors S1 and S2 is connected to, for example, the object terminal 202 and a universal serial bus (USB) via the reading unit 203. More specifically, for example, when the object P attaches each of the sensors S1 and S2 to a USB port of the reading unit 203, the object terminal 202 acquires data from each of the sensors S1 and S2. Note that the plurality of USB ports may be provided in the reading unit 203. Furthermore, the object terminal 202 may include the plurality of reading units 203.

It is assumed that the sensors S1 and S2 be time-synchronized. For example, by recording an absolute time to the sensors S1 and S2 when the sensors S1 and S2 are connected to the object terminal 202, time synchronization may be realized. Note that each of the sensors S1 and S2 may have a wireless communication function. In this case, each of the sensors S1 and S2 may transmit data to the object terminal 202 by wireless communication. Furthermore, the sensors S1 and S2 may be, for example, acceleration sensors that measure accelerations.

Here, an example in which the sensors S1 and S2 are attached will be described. The sensors S1 and S2 can be attached with, for example, a band or tape.

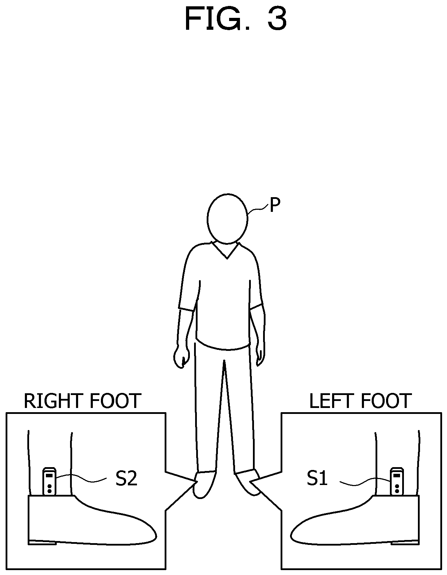

FIG. 3 is an explanatory diagram illustrating an example in which the sensors are attached. As illustrated in FIG. 3, the object P attaches the sensor S1 around the ankle of the left foot and attaches the sensor S2 around the ankle of the right foot. Each of the sensors S1 and S2 measures an angular velocity of each of the plurality of directions and generates inertial data that is expressed by associating a measured value in each direction with a measured time. The object terminal 202 acquires the inertial data generated by the sensors S1 and S2.

Here, an axial direction of the inertial data will be described.

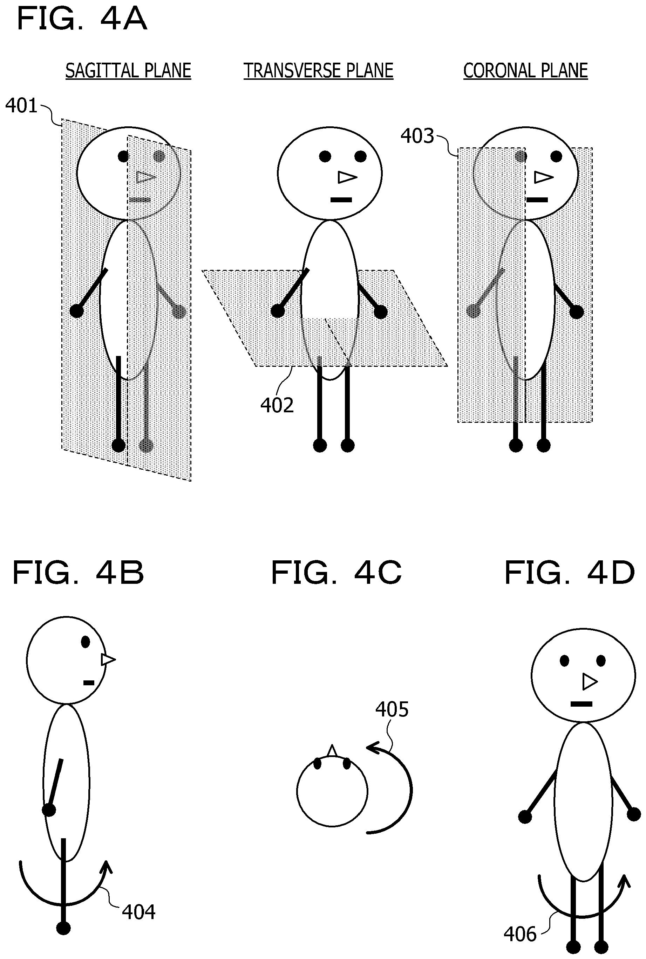

FIGs. 4A to 4D are an explanatory diagram illustrating the axial direction of the inertial data. In FIG. 4A, a sagittal plane 401, a transverse plane 402, and a coronal plane 403 are illustrated. As the axial direction of the inertial data, X-axis gyro data is defined as an angular velocity (rotational velocity) in a sagittal plane direction (front-back direction). Furthermore, Y-axis gyro data is defined as an angular velocity (rotational velocity) in a transverse plane direction (horizontal direction), and Z-axis gyro data is defined as an angular velocity (rotational velocity) in a coronal plane direction (vertical direction).

FIG. 4B illustrates forward rotation (arrow 404) in the front-back direction (sagittal plane direction). FIG. 4C illustrates forward rotation (arrow 405) in the horizontal direction (transverse plane direction). FIG. 4D illustrates forward rotation (arrow 406) in the vertical direction (coronal plane direction). Each of the sensors S1 and S2 measures the X-axis gyro data, the Y-axis gyro data, and the Z-axis gyro data at each fixed time, for example, about 10 milliseconds.

(Exemplary Hardware Configuration of Information Processing Apparatus 101)

Next, an exemplary hardware configuration of the information processing apparatus 101 will be described.

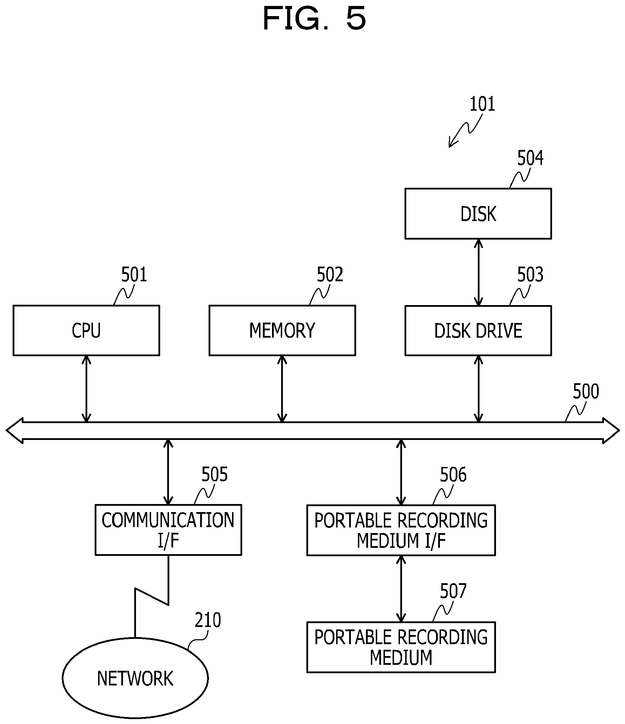

FIG. 5 is a block diagram illustrating an exemplary hardware configuration of the information processing apparatus 101. In FIG. 5, the information processing apparatus 101 includes a central processing unit (CPU) 501, a memory 502, a disk drive 503, a disk 504, a communication interface (I/F) 505, a portable recording medium I/F 506, and a portable recording medium 507. Furthermore, the components are connected to each other via a bus 500.

Here, the CPU 501 performs overall control of the information processing apparatus 101. The CPU 501 may include a plurality of cores. The memory 502 includes, for example, a read only memory (ROM), a random access memory (RAM), a flash ROM, or the like. Specifically, the flash ROM stores operating system (OS) programs, the ROM stores application programs, and the RAM is used as a work area for the CPU 501, for example. A program stored in the memory 502 is loaded into the CPU 501 to cause the CPU 501 to execute coded processing.

The disk drive 503 controls reading and writing of data from and to the disk 504 under the control of the CPU 501. The disk 504 stores data written under the control of the disk drive 503. The disk 504 may be a magnetic disk, an optical disk, or the like, for example.

The communication I/F 505 is connected to the network 210 through a communication line and is connected to an external computer (for example, administrator terminal 201 and object terminal 202 illustrated in FIG. 2) via the network 210. Furthermore, the communication I/F 505 manages an interface between the network 210 and the inside of the device, and controls input and output of data to and from the external computer. For example, a modem, a LAN adapter, or the like can be employed as the communication I/F 505.

The portable recording medium I/F 506 controls reading and writing of data from and into the portable recording medium 507 under the control of the CPU 501. The portable recording medium 507 stores data written under the control of the portable recording medium I/F 506. Examples of the portable recording medium 507 include a compact disc (CD)-ROM, a digital versatile disk (DVD), a universal serial bus (USB) memory, or the like.

Note that the information processing apparatus 101 may include, for example, a solid state drive (SSD), an input device, a display, and the like in addition to the above-described configuration elements. Furthermore, the information processing apparatus 101 may not include, for example, the disk drive 503, the disk 504, the portable recording medium I/F 506, and the portable recording medium 507 among the above-described configuration elements. Furthermore, the administrator terminal 201 and the object terminal 202 illustrated in FIG. 2 can be implemented by a hardware configuration similar to that of the information processing apparatus 101. However, the administrator terminal 201 and the object terminal 202 include, for example, an input device and a display in addition to the above-described configuration elements.

(Storage Content of Inertial Data DB 220)

Next, the storage content of the inertial data DB 220 included in the information processing apparatus 101 will be described. The inertial data DB 220 is implemented by a storage device, for example, the memory 502, the disk 504, or the like of the information processing apparatus 101 illustrated in FIG. 5.

FIG. 6 is an explanatory diagram illustrating an example of the storage content of the inertial data DB 220. In FIG. 6, the inertial data DB 220 includes fields of a patient ID, a time, left foot X-axis gyro, left foot Y-axis gyro, left foot Z-axis gyro, right foot X-axis gyro, right foot Y-axis gyro, and right foot Z-axis gyro. By setting information in each field, gyro data (for example, gyro data 600-1 to 600-7) is stored as a record.

Here, the patient ID is an identifier that uniquely identifies the object P. The time is a measured time of an angular velocity in each axial direction (unit: millisecond). The left foot X-axis gyro indicates an angular velocity in the front-back direction measured by the sensor S1 attached to the left foot of the object P (unit: deg/second). The left foot Y-axis gyro indicates an angular velocity in the horizontal direction measured by the sensor S1 attached to the left foot of the object P (unit: deg/second). The left foot Z-axis gyro indicates an angular velocity in the vertical direction measured by the sensor S1 attached to the left foot of the object P (unit: deg/second).

The right foot X-axis gyro indicates an angular velocity in the front-back direction measured by the sensor S2 attached to the right foot of the object P (unit: deg/second). The right foot Y-axis gyro indicates an angular velocity in the horizontal direction measured by the sensor S2 attached to the right foot of the object P (unit: deg/second). The right foot Z-axis gyro indicates an angular velocity in the vertical direction measured by the sensor S2 attached to the right foot of the object P (unit: deg/second).

The left foot X-axis gyro and the right foot X-axis gyro correspond to the X-axis gyro data described with reference to FIGs. 4A to 4D. The left foot Y-axis gyro and the right foot Y-axis gyro correspond to the Y-axis gyro data described with reference to FIGs. 4A to 4D. The left foot Z-axis gyro and the right foot Z-axis gyro correspond to the Z-axis gyro data described with reference to FIGs. 4A to 4D.

(Exemplary Functional Configuration of Information Processing Apparatus 101)

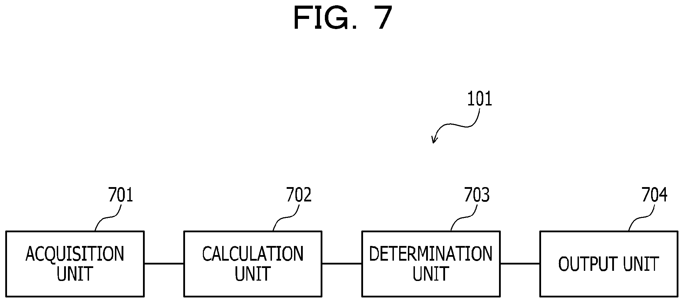

FIG. 7 is a block diagram illustrating an exemplary functional configuration of the information processing apparatus 101. In FIG. 7, the information processing apparatus 101 includes an acquisition unit 701, a calculation unit 702, a determination unit 703, and an output unit 704. Specifically, for example, each functional unit realizes its function by causing the program stored in the storage device such as the memory 502, the disk 504, or the portable recording medium 507 illustrated in FIG. 5 to be executed by the CPU 501 or by the communication I/F 505. A processing result of each functional unit is stored in the storage device, for example, the memory 502, the disk 504, or the like.

The acquisition unit 701 acquires the first measurement data and the second measurement data. Here, the first measurement data indicates the transition of the measured value measured in the predetermined period T by the sensor S1 of the sensors S1 and S2 respectively attached to the left and right feet of the object P. Furthermore, the second measurement data indicates the transition of the measured value measured by the sensor S2 in the predetermined period T.

The predetermined period T is a target period to be determined whether or not the period is a walking period and can be arbitrarily set. A time length of the predetermined period T is set to, for example, a typical time period required for a human to walk about four to six steps. As an example, when the time length is set to "eight seconds", the information processing apparatus 101 may set a period for each eight seconds as the predetermined period T when the health condition of the object P is monitored. However, the periods may be partially overlapped.

Specifically, for example, the acquisition unit 701 acquires gyro data in which the time (measured time) is included in the predetermined period T from the inertial data DB 220 illustrated in FIG. 6. As a result, the first measurement data and the second measurement data can be acquired that respectively indicate the transitions of the measured values measured by the sensors S1 and S2 respectively attached to the left and the right feet of the object P1 in the predetermined period T.

In this case, the first measurement data indicates the transition of the measured value (left foot X-axis gyro, left foot Y-axis gyro, and left foot Z-axis gyro) measured in the predetermined period T by the sensor S1 attached to the left foot of the object P1. The second measurement data indicates the transition of the measured value (right foot X-axis gyro, right foot Y-axis gyro, and right foot Z-axis gyro) measured in the predetermined period T by the sensor S2 attached to the right foot of the object P1.

Furthermore, the predetermined period T may be a period that is estimated as a walking period according to the existing technique, for example, a period determined as a walking section on the basis of the data of the sensors S1 and S2 respectively attached to the left and the right feet of the object P. Note that, as the existing technique for determining the walking section, for example, International Publication Pamphlet No. WO 2017/199305 can be referred.

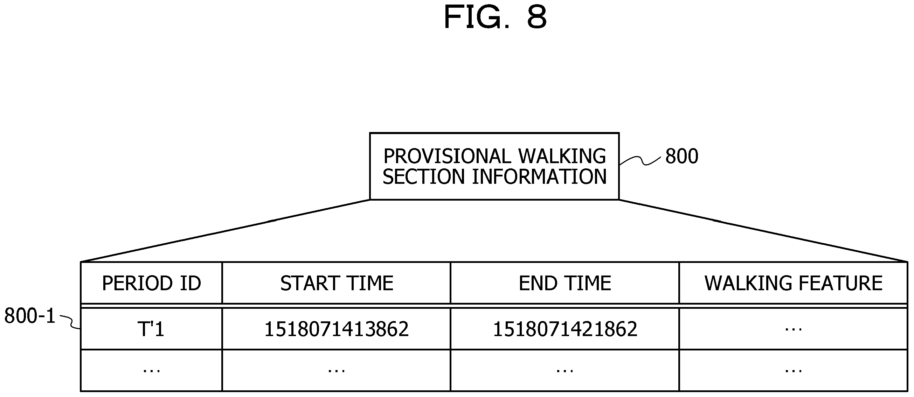

For example, the information processing apparatus 101 may set a provisional walking section as the predetermined period T by referring to provisional walking section information 800 as illustrated in FIG. 8. The provisional walking section is a period determined as the walking section according to the existing technique.

FIG. 8 is an explanatory diagram illustrating a specific example of provisional walking section information. In FIG. 8, the provisional walking section information 800 has fields of a period ID, a start time, an end time, and a walking feature and stores a provisional determination result (for example, provisional determination result 800-1) as a record by setting information in each field.

The period ID is an identifier that uniquely identifies a provisional walking section. The start time is a start time of the provisional walking section (unit: milliseconds). The end time is an end time of the provisional walking section (unit: milliseconds). The walking feature is information indicating a walking feature in the provisional walking section. The walking feature includes, for example, the number of steps, a stride length, a walking speed, a time difference between swings of the left and the right legs, or the like in the provisional walking section. The number of steps is the number of steps during the object P has walked. The walking feature is calculated by using the existing technique (for example, refer to International Publication Pamphlet No. WO 2017/199305) .

For example, the provisional determination result 800-1 indicates a start time "1518071413862", an end time "1518071421862", and a walking feature "..." of a provisional walking section T'1. The information processing apparatus 101 sets the provisional walking section T'1 as the predetermined period T with reference to, for example, the provisional determination result 800-1. In this case, the acquisition unit 701 acquires the first measurement data and the second measurement data by acquiring the gyro data in which the time (measured time) is included in the predetermined period T "1518071413862 to 1518071421862" from the inertial data DB 220.

Note that the provisional walking section information 800 may be acquired from another computer (for example, administrator terminal 201). Furthermore, the provisional walking section information 800 may be generated by the information processing apparatus 101. In other words, the information processing apparatus 101 may generate the provisional walking section information 800 by determining whether or not the predetermined period T is a walking section on the basis of the first measurement data and the second measurement data using the existing technique for determining the walking section.

Returning to the description of FIG. 7, the calculation unit 702 calculates a correlation degree C indicating a degree of a correlation between the transition of the measured value indicated by the acquired first measurement data and the transition of the measured value indicated by the acquired second measurement data. Specifically, for example, the calculation unit 702 can calculate the correlation degree C between the first measurement data and the second measurement data using the following formula (1). However, left foot gyro data at a time t is set to x (t), and right foot gyro data at the time t is set to y (t). Furthermore, the predetermined period T is set to [t1, t2], in other words, a period from the time t1 to the time t2.

[Expression 1]

$$C = \left| \frac{\frac{1}{t2-t1}\sum_{t=t1}^{t2}(x(t)-\overline{x(t)})(y(t)-\overline{y(t)})}{\sqrt{\frac{1}{t2-t1}\sum_{t=t1}^{t2}(x(t)-\overline{x(t)})^2} \times \sqrt{\frac{1}{t2-t1}\sum_{t=t1}^{t2}(y(t)-\overline{y(t)})^2}} \right| \quad ...(1)$$

$\overline{x(t)}$: Average value of x (t) in period [t1, t2]

$\overline{y(t)}$: Average value of y (t) in period [t1, t2]

Note that, in a case where the sensors S1 and S2 can measure angular velocities in a plurality of directions, a direction of the measured value of each of the sensors S1 and S2 to be used can be arbitrarily set when the correlation degree C is obtained. For example, it is assumed that a measured value in the front-back direction (X-axis direction) of each of the sensors S1 and S2, in other words, the X-axis gyro data be set to be used. In this case, the calculation unit 702 calculates the correlation degree C while setting left foot X-axis gyro data at the time t to be x (t) and right foot X-axis gyro data at the time t to be y (t) .

The determination unit 703 determines whether or not walking is performed on the basis of the calculated correlation degree C. Specifically, for example, the determination unit 703 determines whether or not the predetermined period T is a walking period. More specifically, for example, in a case where the correlation degree C is less than a first threshold $\alpha$, the determination unit 703 determines that the predetermined period T is a walking period. At this time, the determination unit 703 may determine that the predetermined period T includes the walking period. On the other hand, in a case where the correlation degree C is equal to or more than the first threshold $\alpha$, the determination unit 703 determines that the predetermined period T is not a walking period. The first threshold $\alpha$ is set to, for example, a value of about 0.2.

With this setting, it can be determined whether or not the predetermined period T is a walking period using the tendency such that the measurement data of the left foot and the measurement data of the right foot have no correlation because, when one foot is swinging at the time of walking, another foot is stopped.

The output unit 704 outputs a determined determination result. Furthermore, the output unit 704 may output the determined determination result in association with the predetermined period T. Specifically, for example, the output unit 704 may output a determination result indicating whether or not the predetermined period T is a walking period for each predetermined period T. Furthermore, the output unit 704 may output information from which a period determined as a walking period can be determined in association with each piece of waveform data that indicates the transition of the measured value measured by each of the sensors S1 and S2.

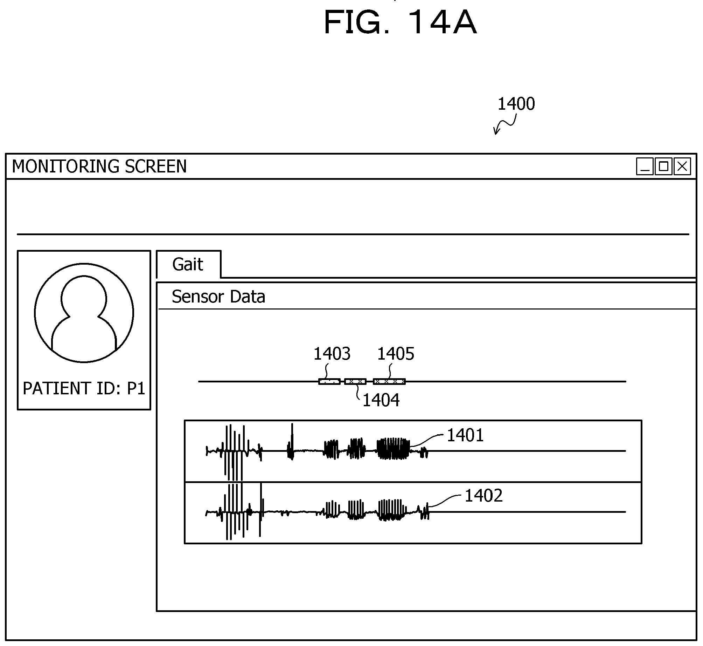

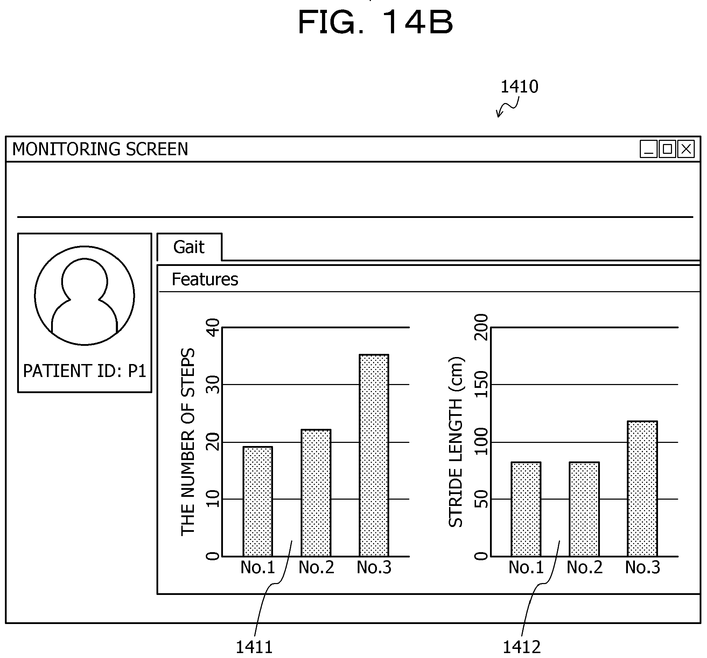

An output format of the output unit 704 includes, for example, storage to a storage device such as the memory 502 or the disk 504, transmission to another computer by the communication I/F 505, print output to a printer (not illustrated), or the like. More specifically, for example, the output unit 704 may display monitoring screens 1400 and 1410 as illustrated in FIGs. 14A and 14B to be described later on the administrator terminal 201.

Note that, in the above description, an example has been described in which the angular velocity is used as the measured value measured by each of the sensors S1 and S2. However, the measured value is not limited to this. For example, an acceleration may be used as the measured value measured by each of the sensors S1 and S2.

(Another Determination Example of Walking Period)

Here, there is a case where the correlation between the measurement data of the sensor S1 and the measurement data of the sensor S2 is lowered due to a factor that the object P changes a way to hold the sensors S1 and S2 while carrying the sensors S1 and S2, for example. In this case, even though the object P is not walking, there is a possibility that this period is wrongly determined as a walking period.

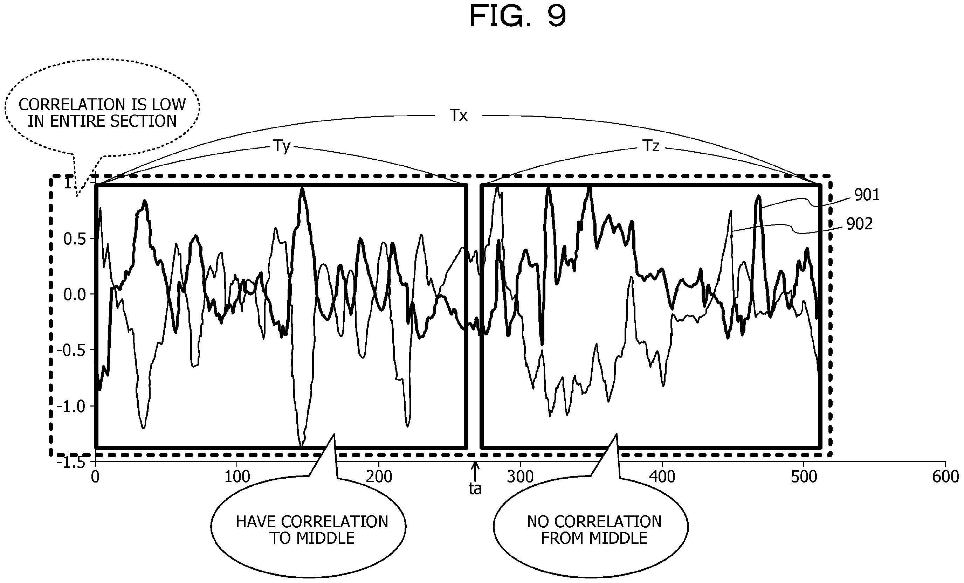

FIG. 9 is an explanatory diagram illustrating a specific example of measurement data at the time of non-walking. In FIG. 9, measurement data 901 indicates a transition of a measured value measured by the sensor S1 in a predetermined period Tx. Measurement data 902 indicates a transition of a measured value measured by the sensor S2 in the predetermined period Tx. However, the horizontal axis indicates a measured time. The vertical axis indicates a value obtained by normalizing the measured value.

Here, the predetermined period Tx is a non-walking period in which the object P carries the sensors S1 and S2 in one's hands, in other words, the object P does not walk in a state where the sensors S1 and S2 are respectively attached to the left and the right feet. Here, a case is assumed where the object P changes the way to hold the sensors S1 and S2 at a time ta when the object P is carrying the sensors S1 and S2.

In this case, in a period Ty before the time ta, the measurement data of the sensor S1 and the measurement data of the sensor S2 have a correlation. On the other hand, in a period Tz after the time ta, the measurement data of the sensor S1 and the measurement data of the sensor S2 have no correlation. As a result, in the entire predetermined period Tx, the correlation between the measurement data of the sensor S1 and the measurement data of the sensor S2 is low.

In this case, when it is determined whether or not the predetermined period Tx is a walking period using the tendency such that the measurement data of the left foot and the measurement data of the right foot have no correlation, this period is wrongly determined as a walking period. However, even in such a case, the correlation is very high in a part of the measurement data 901 and 902.

Therefore, if the correlation is high in a part of the measurement data 901 and 902, the information processing apparatus 101 may determine that the predetermined period Tx is not a walking period even in a case where the correlation is low in the entire predetermined period Tx.

Hereinafter, processing content of each functional unit of the information processing apparatus 101 will be specifically described.

First, the acquisition unit 701 acquires first section data and second section data that respectively indicate transitions of measured values measured by the sensors S1 and S2 for each section having a predetermined time width included in the predetermined period T. The predetermined time width can be arbitrarily set and is set to, for example, about one or two seconds.

In the following description, there is a case where the section having the predetermined time width included in the predetermined period T is expressed as a "window", the predetermined time width is expressed as a "window width", and the section data is expressed as "window data".

Specifically, for example, the acquisition unit 701 acquires first window data and second window data for each window from the first measurement data and the second measurement data while shifting windows in the predetermined period T. The first measurement data indicates a transition of a measured value measured by the sensor S1 in the predetermined period T. The second measurement data indicates a transition of a measured value measured by the sensor S2 in the predetermined period T.

Next, the calculation unit 702 calculates, for each window, a correlation degree C indicating a degree of a correlation between the first window data and the second window data that have been acquired. Specifically, for example, the calculation unit 702 can calculate the correlation degree C between the first window data and the second window data using the above formula (1).

Note that, a direction of a measured value of each of the sensors S1 and S2 to be used when the correlation degree C between the first window data and the second window data is obtained can be arbitrarily set. For example, the calculation unit 702 may calculate the correlation degree C indicating the degree of the correlation between the first window data and the second window data for each combination of directions in which the sensors S1 and S2 can measure angular velocities.

Then, the determination unit 703 determines whether or not the predetermined period T is a walking period on the basis of the calculated correlation degree C for each window. Specifically, for example, the determination unit 703 determines that the predetermined period T is a walking period in a case where the maximum correlation degree C among the calculated correlation degrees C for respective windows is less than a second threshold β. On the other hand, in a case where the maximum correlation degree C is equal to or more than the second threshold β, the determination unit 703 determines that the predetermined period T is not a walking period. The second threshold β is set to, for example, a value of about 0.9.

With this setting, it is possible to determine whether or not the predetermined period T is a walking period, considering that there is a case where the measurement data have no correlation due to the factor that the way to hold the sensors S1 and S2 is changed while the sensors S1 and S2 are carried, for example.

Here, an example for determining whether or not the predetermined period Tx is a walking period will be described using the predetermined period Tx illustrated in FIG. 9 as an example.

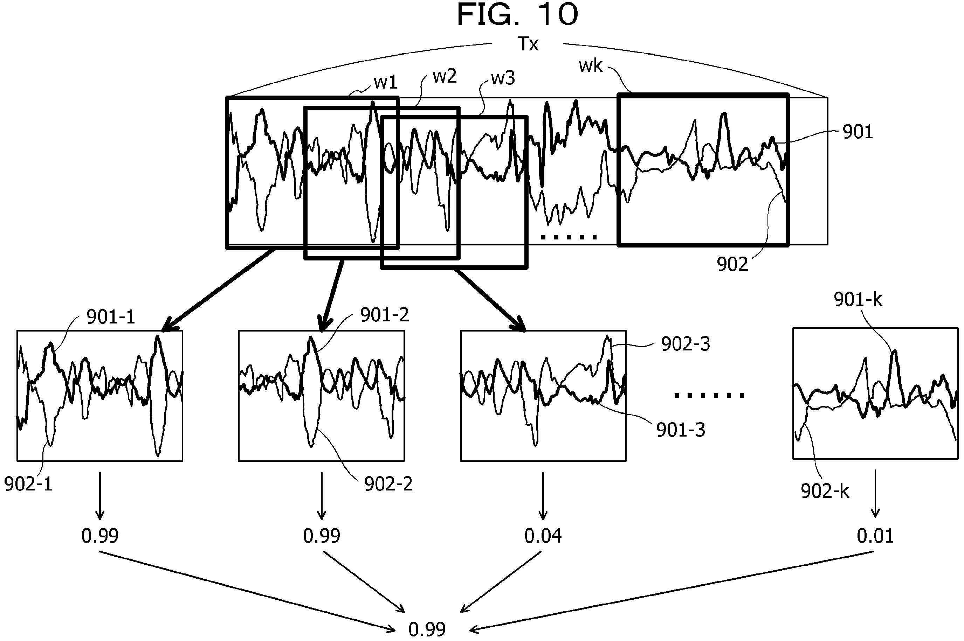

FIG. 10 is an explanatory diagram illustrating a determination example of a walking period. In FIG. 10, windows w1 to wk in the predetermined period Tx are illustrated (k: natural number). In this case, the acquisition unit 701 respectively acquires first window data 901-1 to 901-k and second window data 902-1 to 902-k for the respective windows w1 to wk from the measurement data 901 and 902.

The calculation unit 702 calculates a correlation degree C between the first window data 901-1 to 901-k and the second window data 902-1 to 902-k for each of the windows w1 to wk. Here, it is assumed that "0.99" be calculated as the correlation degree C between the first window data 901-1 and the second window data 902-1 for the window w1.

Furthermore, it is assumed that "0.99" be calculated as the correlation degree C between the first window data 901-2 and the second window data 902-2 for the window w2. Furthermore, it is assumed that "0.04" be calculated as the correlation degree C between the first window data 901-3 and the second window data 902-3 for the window w3. Furthermore, it is assumed that "0.01" be calculated as the correlation degree C between the first window data 901-k and the second window data 902-k for the window wk.

The determination unit 703 determines whether or not the predetermined period Tx is a walking period on the basis of the calculated correlation degree C for each of the windows w1 to wk. Specifically, for example, the determination unit 703 determines the maximum correlation degree C among the correlation degrees C for the windows w1 to wk. Here, the maximum correlation degree C is set to "0.99".

Then, the determination unit 703 determines whether or not the specified maximum correlation degree C is less than the second threshold β. Here, the second threshold β is set to "β = 0.9". In this case, the maximum correlation degree C is equal to or more than the second threshold β. Therefore, the determination unit 703 determines that the predetermined period Tx is not a walking period.

Therefore, even in a case where the correlation in the entire predetermined period Tx is lowered by changing how to hold the sensors S1 and S2 while the sensors S1 and S2 are carried, a non-walking period is correctly removed, and wrong determination can be avoided.

However, if the window width is incorrectly set when the window data is acquired, there is a case where the correlation degree C between the measurement data of the left foot and the measurement data of the right foot at the time of walking cannot be correctly obtained.

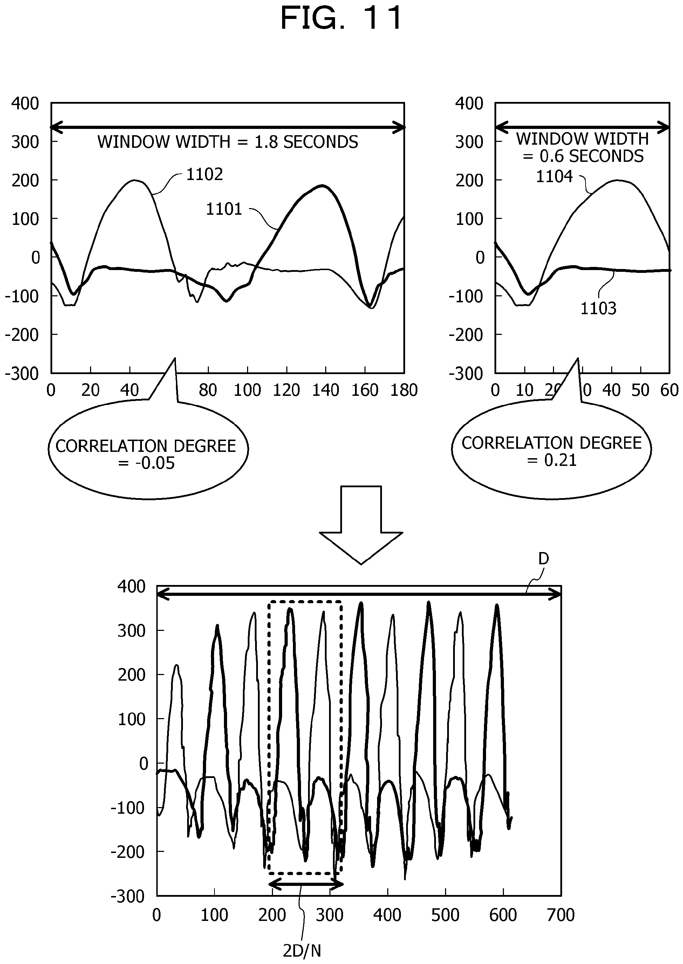

FIG. 11 is an explanatory diagram illustrating a setting example of the window width. In FIG. 11, window data 1101 and 1103 is measurement data at the time of walking and indicates the transition of the measured value measured by the sensor S1. Window data 1102 and 1104 is measurement data at the time of walking and indicates the transition of the measured value measured by the sensor S2. However, the horizontal axis indicates a measured time. The vertical axis indicates a measured value.

Here, in a case where the window width is set to "1.8 seconds", a correlation degree C between the window data 1101 and the window data 1102 is "- 0.05", and it can be said that the correlation degree C at the time of walking can be correctly obtained. On the other hand, in a case where the window width is set to "0.6 seconds", a correlation degree C between the window data 1103 and the window data 1104 is "0.21", and it cannot be said that the correlation degree C at the time of walking is correctly obtained.

In other words, when the window width is set to be too short, a walking cycle is not contained within the window data, and the correlation between the pieces of window data tends to be higher. The walking cycle is a period from a time when the object P starts to swing to a time when the object P starts next swinging. On the other hand, when the window width is set to be too long, an effect of dividing the window data is reduced.

Therefore, the calculation unit 702 may set the window width to a time width that includes at least one or more walking cycles. Specifically, for example, the calculation unit 702 may calculate the window width for the predetermined period T using the following formula (2). However, the predetermined period T is a period that is estimated as a walking section. The reference N indicates the number of steps in the predetermined period T. The reference D indicates a time length of the predetermined period T. The predetermined period T, the number of steps N, and the time length D are specified, for example, from the provisional determination result of the provisional walking section information 800 illustrated in FIG. 8.

$$\text{Window width} = 2D/N \ \ldots \ (2)$$

In this case, the calculation unit 702 sets, for example, the window width to be equal to or more than 2D/N. This makes it possible to prevent that the walking cycle is not included within the window data and to correctly obtain the correlation degree C at the time of walking. Note that the window width may be reset, for example, for each predetermined period T or may be collectively set on the basis of the average number of steps in the predetermined period T or the like.

(Selection of Gyro Data)

Here, a rotational movement when the foot is swinging at the time of walking appears in gyro data in the front-back direction. However, for example, in a case where one leg swings while being bent outward due to a one-leg replacement surgery, an accident, or the like, the rotational movement tends to appear in gyro data in the horizontal direction, not in the gyro data in the front-back direction.

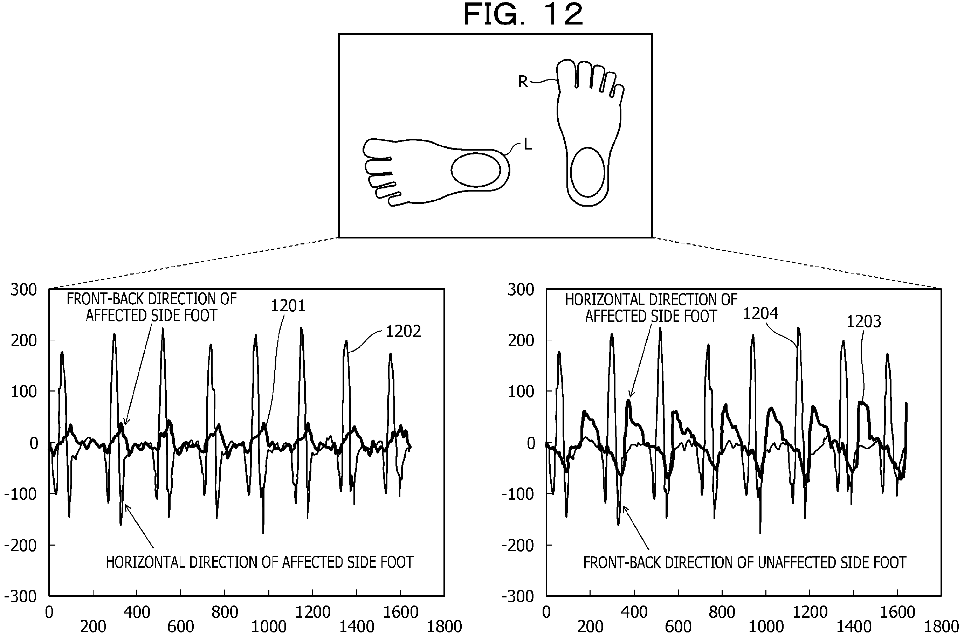

FIG. 12 is an explanatory diagram illustrating a specific example of measurement data at the time of walking with legs flexed 90 degrees. As illustrated in FIG. 12, a case is assumed where the left foot L of the object P (affected side foot) is bent 90 degrees with respect to the right foot R (unaffected side foot). Note that the affected side foot is a physically disabled foot. The unaffected side foot is a foot that is not physically disabled.

In this case, when gyro data 1201 and 1202 that indicates transitions of measured values in the front-back direction for both feet is used, the correlation degree C tends to be higher even at the time of walking. On the other hand, when gyro data 1203 that indicates a transition of a measured value in the horizontal direction is used for the left foot L and gyro data 1204 that indicates a transition of a measured value in the front-back direction is used for the right foot R, the correlation degree C tends to be lower.

Therefore, when the correlation degree C for the predetermined period T is obtained, the calculation unit 702 may determine a direction of the measured value of each of the sensors S1 and S2 to be used. For example, in a case of the unaffected side foot, a motion amount in the front-back direction becomes the largest, and in a case of the affected side foot that is bent 90 degrees with respect to the unaffected side foot, a motion amount in the horizontal direction becomes the largest.

Therefore, the calculation unit 702 may preferentially use the gyro data that indicates the transition of the measured value in the direction in which the amplitude or the peak of the measured value measured by the sensor S1 is large among the plurality of directions of which the angular velocity can be measured as the first measurement data. Furthermore, the calculation unit 702 may preferentially use the gyro data that indicates the transition of the measured value in the direction in which the amplitude or the peak of the measured value measured by the sensor S2 is large

among the plurality of directions of which the angular velocity can be measured as the second measurement data.

In other words, the gyro data in the direction in which the motion amount is large may be used from among the plurality of directions (axial directions) of which the angular velocity can be measured by each of the sensors S1 and S2. Specifically, for example, the calculation unit 702 detects each peak from the gyro data that indicates the transition of the measured value in each direction (front-back direction, horizontal direction, and vertical direction) for the sensor S1.

Next, the calculation unit 702 calculates an average value of the peaks detected from the gyro data in the respective directions. However, the average value of the peaks may be an upper average value of the peaks. The upper average value is an average value using some of upper values. Furthermore, a method for detecting the peak, any existing technique may be used.

Then, the calculation unit 702 specifies a direction in which the average value of the peak is the largest from among the plurality of directions as the direction in which the motion amount is the largest, for the sensor S1. In this case, the calculation unit 702 uses the gyro data in the specified direction as the first measurement data. However, the calculation unit 702 may use gyro data in a direction in which the average value of the peaks is equal to or more than a predetermined threshold.

Similarly, the calculation unit 702 detects each peak from the gyro data that indicates the transition of the measured value in each direction (front-back direction, horizontal direction, and vertical direction) for the sensor S2. Next, the calculation unit 702 calculates an average value of the peaks detected from the gyro data in the respective directions. Then, the calculation unit 702 specifies a direction in which the average value of the peak is the largest from among the plurality of directions as the direction in which the motion amount is the largest, for the sensor S2. In this case, the calculation unit 702 uses the gyro data in the specified direction as the second measurement data.

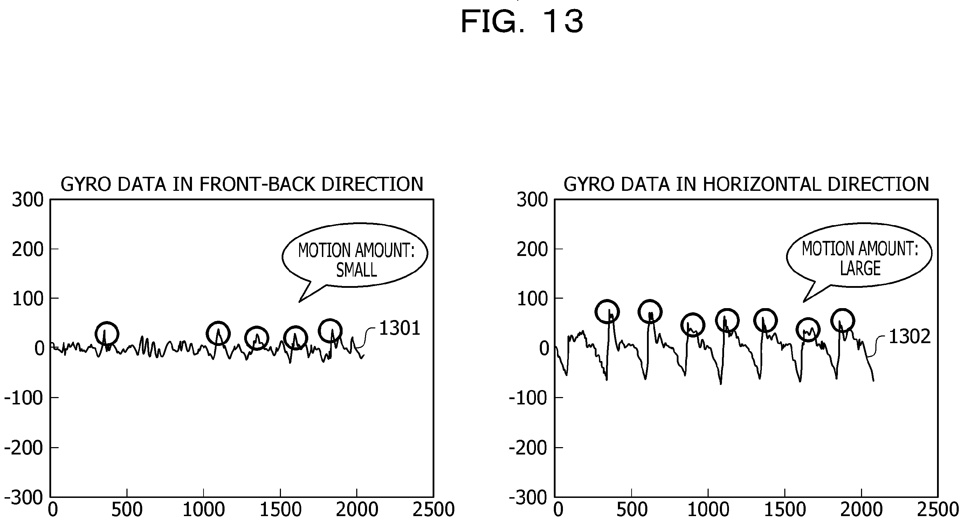

FIG. 13 is an explanatory diagram illustrating a comparative example of peaks of gyro data. In FIG. 13, gyro data 1301 indicates a transition of an angular velocity in the front-back direction for the sensor S1. Gyro data 1302 indicates a transition of an angular velocity in the horizontal direction for the sensor S1.

Here, when an average value of peaks of the gyro data 1301 is compared with an average value of peaks of the gyro data 1302, the average value of the gyro data 1302 is larger. In this case, the calculation unit 702 uses the gyro data 1302 in the horizontal direction having a larger motion amount among those in the respective directions (front-back direction and horizontal direction) as the first measurement data.

Note that, when the direction in which the motion amount is the largest is specified, for example, the calculation unit 702 may specify a direction in which an area obtained by integrating the gyro data is the largest. The area corresponds to an area of a figure surrounded by a waveform indicated by the gyro data and a straight line indicating that the measured value is zero.

(Screen Example of Monitoring Screen)

Next, a screen example of the monitoring screen displayed on the administrator terminal 201 will be described with reference to FIGs. 14A and 14B.

FIGs. 14A and 14B are explanatory diagrams illustrating screen examples of the monitoring screen. In FIG. 14A, the monitoring screen 1400 is an operation screen that displays pieces of information 1403 to 1405 used to specify a period that is determined as a walking period, in association with sensing data 1401 and 1402 regarding the object P1.

The pieces of sensing data 1401 and 1402 are pieces of waveform data that respectively indicate transitions of measured values measured by the sensors S1 and S2 respectively attached to the left and the right feet of the object P1. According to the monitoring screen 1400, a user (for example, doctor) can specify a walking period of the object P1.

Furthermore, in FIG. 14B, the monitoring screen 1410 displays a walking feature in the walking period. The walking feature includes, for example, the number of steps, a stride length, or the like. The walking feature is specified, for example, from the provisional determination result in the provisional walking section information 800.

Here, the number of steps in each walking section is displayed in a graph 1411. Furthermore, a stride length in each walking section is displayed in a graph 1412. The horizontal axis in each graph indicates a number of the walking section. For example, a notation No. 1 in the graph indicates a walking section of the information 1403, a notation No. 2 indicates a walking section of the information 1404, and a notation No. 3 indicates a walking section of the information 1405.

According to the monitoring screen 1410, a user (for example, doctor) can determine a health condition from the walking feature of the object P1. In other words, the health condition of the object P1 can be grasped from a remote place even if the object P1 does not go to a hospital or the doctor does not visit the home of the object P1.

(Walking State Determination Processing Procedure of Information Processing Apparatus 101)

Next, a walking state determination processing procedure of the information processing apparatus 101 will be described. Here, a case where the predetermined period T is set to the provisional walking section that is estimated as a walking period will be described as an example.

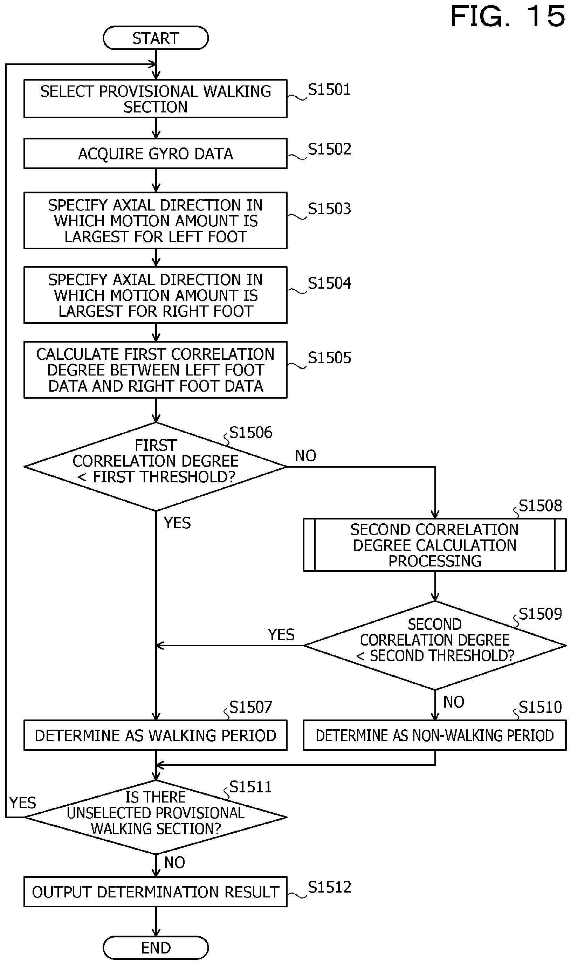

FIG. 15 is a flowchart illustrating an example of the walking state determination processing procedure of the information processing apparatus 101. In the flowchart in FIG. 15, first, the information processing apparatus 101 selects an unselected provisional walking section with reference to the provisional walking section information 800 as illustrated in FIG. 8 (step S1501).

Next, the information processing apparatus 101 acquires gyro data in each axial direction in the selected provisional walking section from the inertial data DB 220 (step S1502). In the following description, there is a case where gyro data of the left foot to which the sensor S1 is attached is expressed as "left foot data" and gyro data of the right foot to which the sensor S2 is attached is expressed as "right foot data".

Next, the information processing apparatus 101 specifies an axial direction in which a motion amount is the largest for the left foot on the basis of the acquired left foot data in each axial direction (step S1503). Next, the information processing apparatus 101 specifies an axial direction in which a motion amount is the largest for the right foot on the basis of the acquired right foot data in each axial direction (step S1504).

Then, the information processing apparatus 101 calculates a correlation degree C between the left foot data in the axial direction, in which the motion amount is the largest, specified in step S1503 and the right foot data in the axial direction, in which the motion amount is the largest, specified in step S1504 (step S1505). In the following description, there is a case where the correlation degree calculated in step S1505 is expressed as a "first correlation degree C1".

Next, the information processing apparatus 101 determines whether or not the calculated first correlation degree C1 is less than the first threshold $\alpha$ (step S1506). Here, in a case where the first correlation degree C1 is less than the first threshold $\alpha$ (step S1506: Yes), the information processing apparatus 101 determines that the selected provisional walking section is a walking period (step S1507), and proceeds the procedure to step S1511.

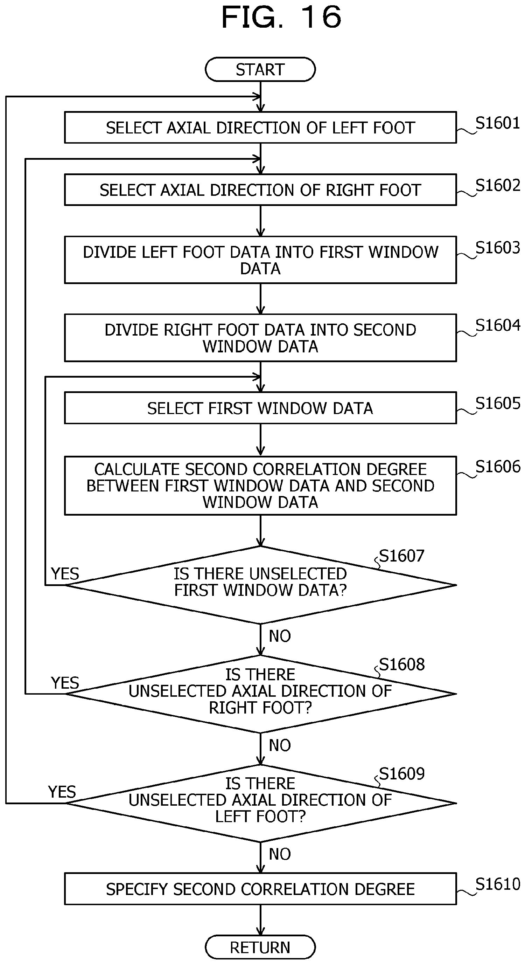

On the other hand, in a case where the first correlation degree C1 is equal to or more than the first threshold $\alpha$ (step S1506: No), the information processing apparatus 101 executes second correlation degree calculation processing for calculating a second correlation degree C2 (step S1508). The second correlation degree C2 corresponds to the maximum correlation degree C among the correlation degrees C for the respective windows described above. A specific processing procedure of the second correlation degree calculation processing will be described later with reference to FIG. 16.

Next, the information processing apparatus 101 determines whether or not the calculated second correlation degree C2 is less than the second threshold $\beta$ (step S1509). Here, in a case where the second correlation degree C2 is less than the second threshold $\beta$ (step S1509: Yes), the information processing apparatus 101 proceeds the procedure to step S1507.

On the other hand, in a case where the second correlation degree C2 is equal to or more than the second threshold $\beta$ (step S1509: No), the information processing apparatus 101 determines that the selected provisional walking section is a non-walking period (step S1510). Then, the information processing apparatus 101 determines whether or not there is an unselected provisional walking section with reference to the provisional walking section information 800 (step S1511).

Here, in a case where there is an unselected provisional walking section (step S1511: Yes), the information processing apparatus 101 returns the procedure to step S1501. On the other hand, in a case where there is no unselected provisional walking section (step S1511: No), the information processing apparatus 101 outputs a determination result that indicates whether or not the provisional walking section is a walking period for each selected provisional walking section (step S1512), and terminates the series of processing in this flowchart.

Next, a specific processing procedure of the second correlation degree calculation processing indicated in step S1508 in FIG. 15 will be described.

FIG. 16 is a flowchart illustrating an example of the specific processing procedure of the second correlation degree calculation processing. In the flowchart in FIG. 16, first, the information processing apparatus 101 selects an unselected axial direction from among a plurality of axial directions for the left foot (step S1601). Next, the information processing apparatus 101 selects an unselected axial direction from among the plurality of axial directions for the right foot (step S1602).

Then, the information processing apparatus 101 divides the left foot data in the axial direction selected in step S1601 into the first window data for each window while shifting the windows in the selected provisional walking section (step S1603). Next, the information processing apparatus 101 divides the right foot data in the axial direction selected in step S1602 into the second window data for each window while shifting the windows in the selected provisional walking section (step S1604).

Then, the information processing apparatus 101 selects an unselected piece of the first window data from among the divided pieces of first window data (step S1605). Next, the information processing apparatus 101 calculates a correlation degree C between the selected first window data and the second window data corresponding to the first window data (step S1606).

Then, the information processing apparatus 101 determines whether or not there is an unselected piece of the first window data from among the divided pieces of the first window data (step S1607). Here, in a case where there is an unselected piece of the first window data (step S1607: Yes), the information processing apparatus 101 returns the procedure to step S1605.

On the other hand, in a case where there is no unselected piece of the first window data (step S1607: No), the information processing apparatus 101 determines whether or not there is an unselected axial direction among the plurality of axial directions for the right foot (step S1608) . Here, in a case where there is an unselected axial direction (step S1608: Yes), the information processing apparatus 101 returns the procedure to step S1602.

On the other hand, in a case where there is no unselected axial direction (step S1608: No), the information processing apparatus 101 determines whether or not there is an unselected axial direction from among the plurality of axial directions for the left foot (step S1609). Here, in a case where there is an unselected axial direction (step S1609: Yes), the information processing apparatus 101 returns the procedure to step S1601.

On the other hand, in a case where there is no unselected axial direction (step S1609: No), the information processing apparatus 101 specifies the maximum correlation degree C among the calculated correlation degrees C as the second correlation degree C2 (step S1610) and returns to the step for calling the second correlation degree calculation processing.

As a result, a non-walking section can be appropriately removed, and accuracy of determination regarding whether or not the object P is walking in the provisional walking section can be improved. For example, the axial direction in which the motion amount is the largest is specified for each of the left and the right feet while assuming a possibility that the foot is bent at the time of walking, and it is possible to obtain a first correlation degree C1 between the left foot data and the right foot data in that axial direction. Furthermore, in the second correlation degree calculation processing in step S1508, in consideration of a possibility that the directions of the sensors S1 and S2 at the time of non-walking are different from each other, the correlation degrees C for all the combinations of the axial directions are obtained, and it is possible to obtain the second correlation degree C2 that is the maximum value of the obtained correlation degrees C.

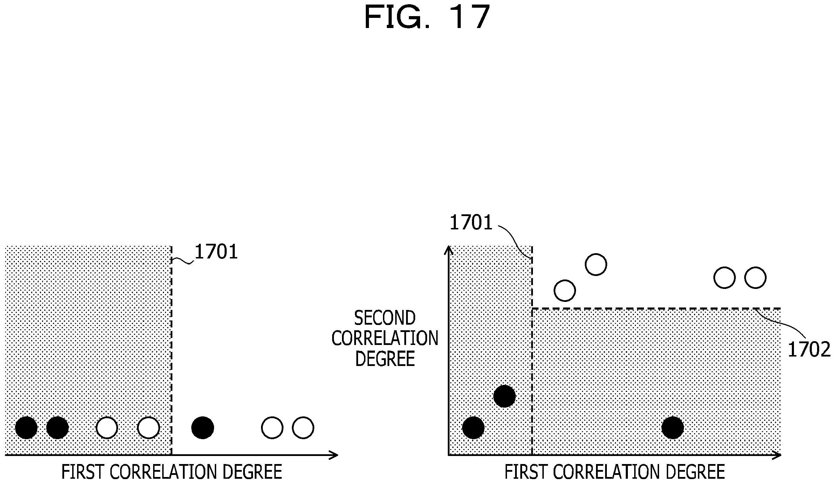

The first correlation degree C1 and the second correlation degree C2 are respectively calculated on the basis of different ideas, and it is possible to improve the walking section determination accuracy by making determination by combining the first correlation degree C1 and the second correlation degree C2. Here, an example of threshold determination based on the first correlation degree C1 and the second correlation degree C2 will be described with reference to FIG. 17.

FIG. 17 is an explanatory diagram illustrating an example of the threshold determination. In FIG. 17, a state of threshold determination using only the first correlation degree C1 is illustrated on the left side, and a state of threshold determination made by combining the first correlation degree C1 and the second correlation degree C2 is illustrated on the right side.

However, a mark • is a value obtained by plotting a value in a true walking section. A mark o is a value obtained by plotting a value in a true non-walking section. A dotted line 1701 indicates a first threshold $\alpha$ regarding the first correlation degree C1. A dotted line 1702 indicates a second threshold $\beta$ regarding the second correlation degree C2.

In the threshold determination using only the first correlation degree C1, in a case where the first correlation degree C1 is less than the first threshold $\alpha$, in other words, in a case where the first correlation degree C1 is in a region on the left side of the dotted line 1701, the period is determined as a walking period. However, the threshold determination using only the first correlation degree C1 may be wrong.

On the other hand, in the threshold determination made by combining the first correlation degree C1 and the second correlation degree C2, in a case where the second correlation degree C1 is less than the second threshold $\beta$, in other words, in a case where the second correlation degree C2 is in a region on the lower side of the dotted line 1702, the period is determined as a walking period. In this way, by performing the threshold determination by combining the first correlation degree C1 and the second correlation degree C2, it is possible to perform more accurate determination.

As described above, according to the information processing apparatus 101 according to the embodiment, it is possible to acquire the first measurement data and the second measurement data that respectively indicate the transitions of the measured values measured by the sensors S1 and S2 respectively attached to the left and the right feet in the predetermined period T. Furthermore, according to the information processing apparatus 101, it is possible to calculate the correlation degree C indicating the degree of the correlation between the transition of the measured value indicated by the acquired first measurement data and the transition of the measured value indicated by the acquired second measurement data and to determine whether or not walking is performed on the basis of the calculated correlation degree C. For example, in a case where the correlation degree C is less than the first threshold $\alpha$, the information processing apparatus 101 determines that the predetermined period T is a walking period. On the other hand, in a case where the correlation degree C is equal to or more than the first threshold $\alpha$, the information processing apparatus 101 determines that the predetermined period T is not a walking period.

As a result, at the time of walking, it is possible to improve the accuracy of the determination regarding whether or not the object P is in a walking state by using the tendency such that the measurement data of the left foot and the measurement data of the right foot have no correlation. For example, when there is no correlation between the measurement data of the left foot and the measurement data of the right foot, it is possible to determine that the predetermined period T is a walking period. Furthermore, for example, when the measurement data of the left foot and the measurement data of the right foot have a correlation, it is determined that the predetermined period T is not a walking period, and it is possible to appropriately remove a non-walking section.

Furthermore, according to the information processing apparatus 101, it is possible to acquire the first section data and the second section data that respectively indicate the transitions of the measured values measured by the sensor S1 and the sensor S2 for each period (window) having the predetermined time width included in the predetermined period T. Moreover, according to the information processing apparatus 101, for each section, it is possible to calculate the correlation degree C indicating the degree of the correlation between the transition of the measured value indicated by the acquired first section data and the transition of the measured value indicated by the acquired second section data. Then, according to the information processing apparatus 101, it is possible to determine whether or not the predetermined period T is a walking period on the basis of the correlation degree C for each calculated section. For example, the information processing apparatus 101 determines that the predetermined period T is a walking period in a case where the maximum correlation degree C among the correlation degrees C for the respective sections is less than the second threshold β. On the other hand, in a case where the maximum correlation degree C is equal to or more than the second threshold β, the information processing apparatus 101 determines that the predetermined period T is not a walking period.

With this setting, it is possible to determine whether or not the predetermined period T is a walking period, considering that there is a case where the measurement data have no correlation due to the factor that the way to hold the sensors S1 and S2 is changed while the sensors S1 and S2 are carried, for example. Therefore, for example, even in a case where the correlation of the entire predetermined period T is lowered by changing the way to hold the sensors S1 and S2 while the sensors S1 and S2 are carried, a non-walking section is correctly removed, and wrong determination can be avoided.

Furthermore, according to the information processing apparatus 101, it is possible to acquire the first section data that indicates the transition of the measured value measured by the sensor S1 for each of the plurality of directions in which the sensors S1 and S2 can measure the angular velocities. Furthermore, according to the information processing apparatus 101, it is possible to acquire the second section data that indicates the transition of the measured value measured by the sensor S2 for each of the plurality of directions in which the sensors S1 and S2 can measure the angular velocities. Then, according to the information processing apparatus 101, for each section included in the predetermined period T, it is possible to calculate the correlation degrees C for all the combinations of the acquired first section data and second section data.

As a result, in consideration of a possibility that the directions of the sensors S1 and S2 at the time of non-walking are different from each other, it is possible to obtain the correlation degrees C for all the combinations of the axial directions and to determine whether or not the period is a walking period from the maximum correlation degree C (second correlation degree C2). Therefore, for example, it is possible to prevent that the correlation between the left section data and the right section data is lowered and the section is wrongly determined as a walking period because the directions of the sensors S1 and S2 are different from each other even in a non-walking state.

Furthermore, according to the information processing apparatus 101, it is possible to set a period (provisional walking period) that is estimated as a walking period as the predetermined period T. With this setting, it is possible to determine that the walking period estimated according to the existing technique different from the present walking state determination method is correct.

Furthermore, according to the information processing apparatus 101, the time width of the section (window) can be set to a time width including at least one or more walking cycles. For example, the information processing apparatus 101 sets the time width of the section (window) on the basis of the time length of the predetermined period T estimated as a walking period and the number of steps in the predetermined period T.

This makes it possible to prevent that the walking cycle is not included within the section data and to correctly obtain the correlation degree C at the time of walking.

Furthermore, according to the information processing apparatus 101, as the first measurement data, the gyro data can be used that indicates the transition of the measured value in the direction, in which the amplitude or the peak of the measured value measured by the sensor S1 is large, among the plurality of directions in which the sensors S1 and S2 can measure the angular velocities. Furthermore, according to the information processing apparatus 101, as the second measurement data, the gyro data can be used that indicates the transition of the measured value in the direction, in which the amplitude or the peak of the measured value measured by the sensor S2 is large, among the plurality of directions in which the sensors S1 and S2 can measure the angular velocities.

As a result, it is possible to obtain the correlation degree C using the gyro data in which the rotational movement when the foot is swinging at the time of walking appropriately appears and to correctly obtain the correlation degree C at the time of walking.

Furthermore, according to the information processing apparatus 101, the determined determination result can be output in association with the predetermined period T. As a result, it is possible to provide information regarding a walking section from which a non-walking section is appropriately removed and to utilize the information in a case where the health condition is grasped from the walking feature of the object P or the like.

Furthermore, according to the information processing apparatus 101, in a case where the correlation degree C between the first measurement data and the second measurement data is equal to or more than the first threshold $\alpha$, it is possible to determine whether or not the predetermined period T is a walking period on the basis of the correlation degree C for each section included in the predetermined period T.

Accordingly, it is possible to determine whether or not the predetermined period T is a walking period by combining two types of correlation degrees C (for example, first correlation degree C1 and second correlation degree C2) obtained on the basis of different ideas, and it is possible to improve the determination accuracy.

From these, according to the information processing apparatus 101, it is possible to improve the accuracy of the determination regarding whether or not the object P is in the walking state and to accurately specify the walking section of the object P. As a result, for example, in a remote monitoring system, when a doctor, a nurse, or the like grasp a health condition of a patient from a remote place, it is possible to specify an accurate walking section and present a walking feature.

Note that the walking state determination method described in the present embodiment can be realized by executing a program that has been prepared in advance by a computer such as a personal computer or a work station. This walking state determination program is recorded on a computer-readable recording medium such as a hard disk, a flexible disk, a compact disk (CD), a digital versatile disc (DVD), or a USB memory, and the walking state determination program is read from the recording medium to be executed by the computer. Furthermore, the present walking state determination program may be distributed via a network such as the Internet.

Furthermore, the information processing apparatus 101 described in the present embodiment can also be implemented by a special-purpose integrated circuit (IC) such as a standard cell or a structured application specific integrated circuit (ASIC) or a programmable logic device (PLD) such as a field-programmable gate array (FPGA).

REFERENCE SIGNS LIST

| | |
|---|---|
| 101 | information processing apparatus |
| 102 | first sensor |
| 103 | second sensor |
| 111, 121 | first measurement data |
| 112, 122 | second measurement data |
| 200 | information processing system |
| 201 | administrator terminal |
| 202 | object terminal |
| 203 | reading unit |
| 210 | network |
| 220 | inertial data DB |
| 401 | sagittal plane |
| 402 | transverse plane |
| 403 | coronal plane |
| 404, 405, 406 | arrow |
| 500 | bus |
| 501 | CPU |
| 502 | memory |
| 503 | disk drive |
| 504 | disk |
| 505 | communication I/F |
| 506 | portable recording medium I/F |
| 507 | portable recording medium |
| 701 | acquisition unit |
| 702 | calculation unit |
| 703 | determination unit |
| 704 | output unit |
| 800 | provisional walking section information |
| 901, 902 | measurement data |
| 901-1 to 901-k | first window data |
| 902-1 to 902-k | second window data |

| 1101, 1102, 1103, 1104 | window data |
| 1201, 1202, 1203, 1204, 1301, 1302 | gyro data |
| 1400 | monitoring screen |
| 1401, 1402 | sensing data |
| 1403, 1404, 1405 | information |
| 1410 | details field |
| 1411 | walking feature |
| 1412, 1413 | waveform data |
| 1701, 1702 | dotted line |
| P | object |
| w1 to wk | window |

**Claims**

1. A walking state determination program for causing a computer to execute processing comprising:
setting a predetermined period (Tx) as provisional walking section;

acquiring first measurement data and second measurement data for a plurality of time windows (w1, ..., wk) in the predetermined period (Tx) that respectively indicate transitions of measured values measured by a first sensor and a second sensor respectively attached to a left and a right feet in the predetermined period (Tx);
calculating a correlation degree for each time window (w1, ..., wk) that indicates a degree of a correlation between the transition of the measured value indicated by the acquired first measurement data and the transition of the measured value indicated by the acquired second measurement data;
determining whether or not walking is performed during the predetermined period (Tx) on the basis of the maximum calculated correlation degree among the correlation degrees for the plurality of time windows (w1, ..., wk); and
removing the predetermined period (Tx) during which no walking is performed as a non-walking section from the provisional walking section.

2. The walking state determination program according to claim 1, wherein

the determining processing
determines whether or not the predetermined period (Tx) is a walking period on the basis of the calculated correlation degree.

3. The walking state determination program according to claim 2, wherein

the determining processing
determines that the predetermined period (Tx) is a walking period in a case where the correlation degree is less than a first threshold.

4. The walking state determination program for causing the computer to execute processing according to claim 2 or 3, further comprising:

acquiring first section data and second section data that respectively indicate the transitions of the measured values measured by the first sensor and the second sensor for each section that has a predetermined time width included in the predetermined period (Tx);
calculating a correlation degree that indicates a degree of a correlation between the transition of the measured value indicated by the acquired first section data and the transition of the measured value indicated by the acquired second section data, for each section; and
determining whether or not the predetermined period (Tx) is a walking period on the basis of the calculated correlation degree for each section.

5. The walking state determination program according to claim 4, wherein

the determining processing
determines that the predetermined period (Tx) is a walking period in a case where a maximum correlation

17

degree among the calculated correlation degrees for the respective sections is less than a second threshold.

6. The walking state determination program according to claim 4 or 5, wherein

the predetermined period (Tx) is a period that is estimated as a walking period, and
the predetermined time width is set to a time width that includes at least one or more walking cycles.

7. The walking state determination program according to any one of claims 2 to 6, wherein

the first sensor and the second sensor enable to measure an angular velocity or an acceleration in each of a plurality of directions,
the first measurement data indicates a transition of a measured value in a direction in which an amplitude or a peak of the measured value measured by the first sensor is large among the plurality of directions, and
the second measurement data indicates a transition of a measured value in a direction in which an amplitude or a peak of the measured value measured by the second sensor is large among the plurality of directions.

8. The walking state determination program for causing the computer to execute processing according to any one of claims 2 to 7, further comprising:
outputting a determined determination result in association with the predetermined period (Tx).

9. The walking state determination program according to claim 4, wherein

the determining processing
determines whether or not the predetermined period (Tx) is a walking period on the basis of the calculated correlation degree for each section in a case where the correlation degree is equal to or more than a first threshold.

10. The walking state determination program according to claim 4 or 5, wherein

the first sensor and the second sensor enable to measure an angular velocity or an acceleration in each of a plurality of directions,
the first section data indicates the transition of the measured value measured by the first sensor in each of the plurality of directions,
the second section data indicates the transition of the measured value measured by the second sensor in each of the plurality of directions, and
the processing for calculating the correlation degree for each section
calculates correlation degrees for all combinations of the first section data and the second section data for each section.

11. A walking state determination method for causing a computer to execute processing comprising:

setting a predetermined period (Tx) as provisional walking section;
acquiring first measurement data and second measurement data for a plurality of time windows (w1, ..., wk) in the predetermined period (Tx) that respectively indicate transitions of measured values measured by a first sensor and a second sensor respectively attached to a left and a right feet in the predetermined period (Tx);
calculating a correlation degree for each time window (w1, ..., wk) that indicates a degree of a correlation between the transition of the measured value indicated by the acquired first measurement data and the transition of the measured value indicated by the acquired second measurement data; and
determining whether or not walking is performed during the predetermined period (Tx) on the basis of the maximum calculated correlation degree among the correlation degrees for the plurality of time windows (w1, ..., wk); and
removing the predetermined period (Tx) during which no walking is performed as a non-walking section from the provisional walking section.

12. An information processing apparatus (101) comprising:

the information processing apparatus (101) configured to set a predetermined period (Tx) as provisional walking section;
an acquisition unit (701) configured to acquire first measurement data and second measurement data for a plurality of time windows (w1, ..., wk) in the predetermined period (Tx) that respectively indicate transitions of measured values measured by a first sensor and a second sensor respectively attached to a left and a right feet in the predetermined period (Tx);

a calculation unit (702) configured to calculate a correlation degree for each time window (w1, ..., wk) that indicates a degree of a correlation between the transition of the measured value indicated by the first measurement data and the transition of the measured value indicated by the second measurement data that have been acquired by the acquisition unit; and

a determination unit (703) configured to determine whether or not walking is performed during the predetermined period (Tx) on the basis of the maximum correlation degree calculated by the calculation unit among the correlation degrees for the plurality of time windows (w1,...,wk); and

the information processing apparatus (101) configured to remove the predetermined period (Tx) during which no walking is performed as a non-walking section from the provisional walking section.

**Patentansprüche**

1. Programm zur Bestimmung eines Gehzustands, um einen Computer zu veranlassen, eine Verarbeitung auszuführen, umfassend:

   Festlegen eines vorbestimmten Zeitraums (Tx) als vorläufigen Gehabschnitt;

   Erfassen erster Messdaten und zweiter Messdaten für eine Vielzahl von Zeitfenstern (w1, ..., wk) in dem vorbestimmten Zeitraum (Tx), die jeweils Übergänge von Messwerten angeben, die von einem ersten Sensor und einem zweiten Sensor, die an einem linken bzw. einem rechten Fuß angebracht sind, in dem vorbestimmten Zeitraum (Tx) gemessen werden;

   Berechnen eines Korrelationsgrades für jedes Zeitfenster (w1, ..., wk), der einen Grad einer Korrelation zwischen dem Übergang des Messwerts, der durch die erfassten ersten Messdaten angegeben ist, und dem Übergang des Messwerts, der durch die erfassten zweiten Messdaten angegeben ist, angibt;

   Bestimmen auf der Grundlage des maximalen berechneten Korrelationsgrades unter den Korrelationsgraden für die Vielzahl von Zeitfenstern (w1, ..., wk), ob während des vorbestimmten Zeitraums (Tx) Gehen durchgeführt wird oder nicht; und

   Entfernen des vorbestimmten Zeitraums (Tx), während dessen kein Gehen durchgeführt wird, als Nicht-Gehabschnitt aus dem vorläufigen Gehabschnitt.

2. Programm zur Bestimmung eines Gehzustands nach Anspruch 1, wobei

   die Bestimmungsverarbeitung
   auf der Grundlage des berechneten Korrelationsgrades bestimmt, ob der vorbestimmte Zeitraum (Tx) ein Gehzeitraum ist oder nicht.

3. Programm zur Bestimmung eines Gehzustands nach Anspruch 2, wobei

   die Bestimmungsverarbeitung
   in einem Fall, in dem der Korrelationsgrad kleiner als ein erster Schwellenwert ist, bestimmt, dass der vorbestimmte Zeitraum (Tx) ein Gehzeitraum ist.

4. Programm zur Bestimmung eines Gehzustands, um den Computer zu veranlassen, eine Verarbeitung nach Anspruch 2 oder 3 auszuführen, weiter umfassend:

   Erfassen von ersten Abschnittsdaten und zweiten Abschnittsdaten, die jeweils die Übergänge der Messwerte angeben, die von dem ersten Sensor und dem zweiten Sensor für jeden Abschnitt gemessen werden, der eine vorbestimmte Zeitbreite aufweist, die in dem vorbestimmten Zeitraum (Tx) enthalten ist;

   Berechnen eines Korrelationsgrades, der einen Grad einer Korrelation zwischen dem Übergang des Messwerts, der durch die erfassten ersten Abschnittsdaten angegeben ist, und dem Übergang des Messwerts, der durch die erfassten zweiten Abschnittsdaten angegeben ist, für jeden Abschnitt angibt; und

   Bestimmen auf der Grundlage des berechneten Korrelationsgrades für jeden Abschnitt, ob der vorbestimmte Zeitraum (Tx) ein Gehzeitraum ist oder nicht.

5. Programm zur Bestimmung eines Gehzustands nach Anspruch 4, wobei

die Bestimmungsverarbeitung
in einem Fall, in dem ein maximaler Korrelationsgrad unter den berechneten Korrelationsgraden für die jeweiligen Abschnitte kleiner als ein zweiter Schwellenwert ist, bestimmt, dass der vorbestimmte Zeitraum (Tx) ein Gehzeitraum ist.

6. Programm zur Bestimmung eines Gehzustands nach Anspruch 4 oder 5, wobei

der vorbestimmte Zeitraum (Tx) ein Zeitraum ist, der als ein Gehzeitraum geschätzt wird, und
die vorbestimmte Zeitbreite auf eine Zeitbreite eingestellt ist, die mindestens einen oder mehrere Gehzyklen einschließt.

7. Programm zur Bestimmung eines Gehzustands nach einem der Ansprüche 2 bis 6, wobei

der erste Sensor und der zweite Sensor ermöglichen, eine Winkelgeschwindigkeit oder eine Beschleunigung in jeder von einer Vielzahl von Richtungen zu messen,
die ersten Messdaten einen Übergang eines Messwerts in eine Richtung angeben, in der eine Amplitude oder ein Spitzenwert des von dem ersten Sensor gemessenen Messwerts unter der Vielzahl von Richtungen groß ist, und
die zweiten Messdaten einen Übergang eines Messwerts in eine Richtung angeben, in der eine Amplitude oder ein Spitzenwert des von dem zweiten Sensor gemessenen Messwerts unter der Vielzahl von Richtungen groß ist.

8. Programm zur Bestimmung eines Gehzustands, um den Computer zu veranlassen, eine Verarbeitung nach einem der Ansprüche 2 bis 7 auszuführen, weiter umfassend:
Ausgeben eines bestimmten Bestimmungsergebnisses in Verbindung mit dem vorbestimmten Zeitraum (Tx).

9. Programm zur Bestimmung eines Gehzustands nach Anspruch 4, wobei

die Bestimmungsverarbeitung
in einem Fall, in dem der Korrelationsgrad gleich oder größer als ein erster Schwellenwert ist, auf der Grundlage des berechneten Korrelationsgrades für jeden Abschnitt bestimmt, ob der vorbestimmte Zeitraum (Tx) ein Gehzeitraum ist oder nicht.

10. Programm zur Bestimmung eines Gehzustands nach Anspruch 4 oder 5, wobei

der erste Sensor und der zweite Sensor ermöglichen, eine Winkelgeschwindigkeit oder eine Beschleunigung in jeder von einer Vielzahl von Richtungen zu messen,
die ersten Abschnittsdaten den Übergang des Messwerts angeben, der von dem ersten Sensor in jeder der Vielzahl von Richtungen gemessen wird,
die zweiten Abschnittsdaten den Übergang des Messwerts angeben, der von dem zweiten Sensor in jeder der Vielzahl von Richtungen gemessen wird, und
die Verarbeitung zum Berechnen des Korrelationsgrades für jeden Abschnitt
Korrelationsgrade für alle Kombinationen der ersten Abschnittsdaten und der zweiten Abschnittsdaten für jeden Abschnitt berechnet.

11. Verfahren zur Bestimmung eines Gehzustands, um einen Computer zu veranlassen, eine Verarbeitung auszuführen, umfassend:

Festlegen eines vorbestimmten Zeitraums (Tx) als vorläufigen Gehabschnitt;
Erfassen erster Messdaten und zweiter Messdaten für eine Vielzahl von Zeitfenstern (w1, ..., wk) in dem vorbestimmten Zeitraum (Tx), die jeweils Übergänge von Messwerten angeben, die von einem ersten Sensor und einem zweiten Sensor, die an einem linken bzw. einem rechten Fuß angebracht sind, in dem vorbestimmten Zeitraum (Tx) gemessen werden;
Berechnen eines Korrelationsgrades für jedes Zeitfenster (w1, ..., wk), der einen Grad einer Korrelation zwischen dem Übergang des Messwerts, der durch die erfassten ersten Messdaten angegeben ist, und dem Übergang des Messwerts, der durch die erfassten zweiten Messdaten angegeben ist, angibt; und
Bestimmen auf der Grundlage des maximalen berechneten Korrelationsgrades unter den Korrelationsgraden für die Vielzahl von Zeitfenstern (w1, ..., wk), ob während des vorbestimmten Zeitraums (Tx) Gehen durchgeführt wird oder nicht; und

Entfernen des vorbestimmten Zeitraums (Tx), während dessen kein Gehen durchgeführt wird, als Nicht-Gehabschnitt aus dem vorläufigen Gehabschnitt.

12. Informationsverarbeitungseinrichtung (101), umfassend:

die Informationsverarbeitungsvorrichtung (101), die konfiguriert ist, um einen vorbestimmten Zeitraum (Tx) als vorläufigen Gehabschnitt einzustellen;
eine Erfassungseinheit (701), die konfiguriert ist, um erste Messdaten und zweite Messdaten für eine Vielzahl von Zeitfenstern (w1, ..., wk) in dem vorbestimmten Zeitraum (Tx) zu erfassen, die jeweils Übergänge von Messwerten angeben, die von einem ersten Sensor und einem zweiten Sensor, die an einem linken bzw. einem rechten Fuß angebracht sind, in dem vorbestimmten Zeitraum (Tx) gemessen werden;
eine Berechnungseinheit (702), die konfiguriert ist, um einen Korrelationsgrad für jedes Zeitfenster (w1, ..., wk) zu berechnen, der einen Grad einer Korrelation zwischen dem Übergang des Messwerts, der durch die erfassten ersten Messdaten angegeben ist, und dem Übergang des Messwerts, der durch die erfassten zweiten Messdaten angegeben ist, angibt; und
eine Bestimmungseinheit (703), die konfiguriert ist, um auf der Grundlage des maximalen berechneten Korrelationsgrades, der von der Berechnungseinheit berechnet wird, unter den Korrelationsgraden für die Vielzahl von Zeitfenstern (w1, ..., wk) zu bestimmen, ob während des vorbestimmten Zeitraums (Tx) Gehen durchgeführt wird oder nicht; und
wobei die Informationsverarbeitungsvorrichtung (101) konfiguriert ist, um den vorbestimmten Zeitraum (Tx), während dessen kein Gehen durchgeführt wird, als einen Nicht-Gehabschnitt aus dem vorläufigen Gehabschnitt zu entfernen.

**Revendications**

1. Programme de détermination de l'état de marche pour amener un ordinateur à exécuter un traitement comprenant :
la définition d'une période prédéterminée (Tx) comme section de marche provisoire ;

l'obtention de premières données de mesure et de secondes données de mesure pour une pluralité de fenêtres temporelles (w1, ..., wk) dans la période prédéterminée (Tx) qui indiquent respectivement des transitions de valeurs mesurées mesurées par un premier capteur et un second capteur fixés respectivement à un pied droit et un pied gauche dans la période prédéterminée (Tx) ;
le calcul d'un degré de corrélation pour chaque fenêtre temporelle (w1, ..., wk) qui indique un degré d'une corrélation entre la transition de la valeur mesurée indiquée par les premières données de mesure obtenues et la transition de la valeur mesurée indiquée par les secondes données de mesure obtenues ;
le fait de déterminer si une marche est effectuée ou non pendant la période prédéterminée (Tx) en fonction du degré de corrélation maximal calculé parmi les degrés de corrélation pour la pluralité de fenêtres temporelles (w1, ..., wk) ; et
la suppression de la période prédéterminée (Tx) pendant laquelle aucune marche n'est effectuée comme section de non marche de la section de marche provisoire.

2. Programme de détermination de l'état de marche selon la revendication 1, dans lequel

le traitement de détermination
détermine si la période prédéterminée (Tx) est une période de marche ou non en fonction du degré de corrélation calculé.

3. Programme de détermination de l'état de marche selon la revendication 2, dans lequel

le traitement de détermination
détermine que la période prédéterminée (Tx) est une période de marche dans le cas où le degré de corrélation est inférieur à un premier seuil.

4. Programme de détermination de l'état de marche pour amener l'ordinateur à exécuter un traitement selon la revendication 2 ou 3, comprenant en outre :

l'obtention de premières données de section et de secondes données de section qui indiquent respectivement les transitions des valeurs mesurées mesurées par le premier capteur et le second capteur pour chaque section qui présente une durée prédéterminée incluse dans la période prédéterminée (Tx) ;

le calcul d'un degré de corrélation qui indique un degré d'une corrélation entre la transition de la valeur mesurée indiquée par les premières données de section obtenues et la transition de la valeur mesurée indiquée par les secondes données de section obtenues, pour chaque section ; et

le fait de déterminer si la période prédéterminée (Tx) est une période de marche ou non en fonction du degré de corrélation calculé pour chaque section.

5. Programme de détermination de l'état de marche selon la revendication 4, dans lequel

le traitement de détermination

détermine que la période prédéterminée (Tx) est une période de marche dans le cas où un degré de corrélation maximal parmi les degrés de corrélation calculés pour les sections respectives est inférieur à un second seuil.

6. Programme de détermination de l'état de marche selon la revendication 4 ou 5, dans lequel

la période prédéterminée (Tx) est une période qui est estimée comme une période de marche, et
la durée prédéterminée est définie sur une durée qui inclut au moins un ou plusieurs cycles de marche.

7. Programme de détermination de l'état de marche selon l'une quelconque des revendications 2 à 6, dans lequel

le premier capteur et le second capteur permettent de mesurer une vitesse angulaire ou une accélération dans chacune d'une pluralité de directions,
les premières données de mesure indiquent une transition d'une valeur mesurée dans une direction dans laquelle une amplitude ou un pic de la valeur mesurée mesurée par le premier capteur est important(e) parmi la pluralité de directions, et
les secondes données de mesure indiquent une transition d'une valeur mesurée dans une direction dans laquelle une amplitude ou un pic de la valeur mesurée mesurée par le second capteur est important(e) parmi la pluralité de directions.

8. Programme de détermination de l'état de marche pour amener l'ordinateur à exécuter un traitement selon l'une quelconque des revendications 2 à 7, comprenant en outre :
la production d'un résultat de détermination déterminé en association avec la période prédéterminée (Tx).

9. Programme de détermination de l'état de marche selon la revendication 4, dans lequel

le traitement de détermination

détermine si la période prédéterminée (Tx) est une période de marche ou non en fonction du degré de corrélation calculé pour chaque section dans le cas où le degré de corrélation est supérieur ou égal à un premier seuil.

10. Programme de détermination de l'état de marche selon la revendication 4 ou 5, dans lequel

le premier capteur et le second capteur permettent de mesurer une vitesse angulaire ou une accélération dans chacune d'une pluralité de directions,
les premières données de section indiquent la transition de la valeur mesurée mesurée par le premier capteur dans chacune de la pluralité de directions,
les secondes données de section indiquent la transition de la valeur mesurée mesurée par le second capteur dans chacune de la pluralité de directions, et
le traitement pour le calcul du degré de corrélation pour chaque section
calcule les degrés de corrélation de toutes les combinaisons des premières données de section et des secondes données de section pour chaque section.

11. Programme de détermination de l'état de marche pour amener un ordinateur à exécuter un traitement comprenant :

la définition d'une période prédéterminée (Tx) comme section de marche provisoire ;
l'obtention de premières données de mesure et de secondes données de mesure pour une pluralité de fenêtres temporelles (w1, ..., wk) dans la période prédéterminée (Tx) qui indiquent respectivement des transitions de valeurs mesurées mesurées par un premier capteur et un second capteur fixés respectivement à un pied droit et un pied gauche dans la période prédéterminée (Tx) ;

le calcul d'un degré de corrélation pour chaque fenêtre temporelle (w1, ..., wk) qui indique un degré d'une corrélation entre la transition de la valeur mesurée indiquée par les premières données de mesure obtenues et la transition de la valeur mesurée indiquée par les secondes données de mesure obtenues ; et

le fait de déterminer si une marche est effectuée ou non pendant la période prédéterminée (Tx) en fonction du degré de corrélation maximal calculé parmi les degrés de corrélation pour la pluralité de fenêtres temporelles (w1, ..., wk) ; et

la suppression de la période prédéterminée (Tx) pendant laquelle aucune marche n'est effectuée comme section de non marche de la section de marche provisoire.

12. Appareil de traitement d'informations (101) comprenant :

l'appareil de traitement d'informations (101) configuré pour définir une période prédéterminée (Tx) comme section de marche provisoire ;

une unité d'acquisition (701) configurée pour obtenir des premières données de mesure et des secondes données de mesure pour une pluralité de fenêtres temporelles (w1, ..., wk) dans la période prédéterminée (Tx) qui indiquent respectivement des transitions de valeurs mesurées mesurées par un premier capteur et un second capteur fixés respectivement à un pied droit et un pied gauche dans la période prédéterminée (Tx) ;

une unité de calcul (702) configurée pour calculer un degré de corrélation pour chaque fenêtre temporelle (w1, ..., wk) qui indique un degré d'une corrélation entre la transition de la valeur mesurée indiquée par les premières données de mesure et la transition de la valeur mesurée indiquée par les secondes données de mesure qui ont été obtenues par l'unité d'acquisition ; et

une unité de détermination (703) configurée pour déterminer si une marche est effectuée ou non pendant la période prédéterminée (Tx) en fonction du degré de corrélation maximal calculé par l'unité de calcul parmi les degrés de corrélation pour la pluralité de fenêtres temporelles (w1, ..., wk) ; et

l'appareil de traitement d'informations (101) configuré pour supprimer la période prédéterminée (Tx) pendant laquelle aucune marche n'est effectuée comme section de non marche de la section de marche provisoire.

# FIG. 1

<AT THE TIME OF WALKING>

<AT THE TIME OF NON-WALKING>

LIFT, PUT

CORRELATION DEGREE 「0.03」
→NO CORRELATION
DETERMINATION RESULT: WALKING

CORRELATION DEGREE 「0.99」
→WITH CORRELATION
DETERMINATION RESULT: NOT WALKING

# FIG. 2

200

101

220

INERTIAL DATA DB

210

201

202

S1,S2

203

# FIG. 3

# FIG. 4A

SAGITTAL PLANE   TRANSVERSE PLANE   CORONAL PLANE

401

403

402

# FIG. 4B

404

# FIG. 4C

405

# FIG. 4D

406

# FIG. 5

# FIG. 6

INERTIAL DATA DB ~ 220

| PATIENT ID | TIME (MILLISECOND) | LEFT FOOT X-AXIS GYRO (deg/SECOND) | LEFT FOOT Y-AXIS GYRO (deg/SECOND) | LEFT FOOT Z-AXIS GYRO (deg/SECOND) | RIGHT FOOT X-AXIS GYRO (deg/SECOND) | RIGHT FOOT Y-AXIS GYRO (deg/SECOND) | RIGHT FOOT Z-AXIS GYRO (deg/SECOND) |
|---|---|---|---|---|---|---|---|
| ... | ... | ... | ... | ... | ... | ... | ... |
| P1 | 1518071413862 | 7.776034 | -66.262 | 14.19971 | 27.15903 | -172.818 | 8.957918 |
| P1 | 1518071413871 | 9.277262 | -18.4598 | 29.66482 | -1.90557 | -151.003 | 54.23844 |
| P1 | 1518071413881 | -24.5243 | 19.78022 | -25.5505 | 22.58306 | -233.425 | -65.4614 |
| P1 | 1518071413891 | -20.7064 | 16.66995 | 9.982099 | 50.88229 | -210.952 | -116.106 |
| P1 | 1518071413901 | -15.1534 | 8.350865 | 35.53905 | 51.14353 | -160.831 | -87.8099 |
| P1 | 1518071413910 | -12.5294 | -6.84048 | 30.49709 | 76.23 | -140.617 | -72.6524 |
| P1 | 1518071413920 | -3.48163 | -2.02213 | 44.64048 | 98.89906 | -101.86 | -54.4488 |
| ... | ... | ... | ... | ... | ... | ... | ... |

600-1, 600-2, 600-7

EP 3 847 961 B1

# FIG. 7

101

| 701 | 702 | 703 | 704 |
|---|---|---|---|
| ACQUISITION UNIT | CALCULATION UNIT | DETERMINATION UNIT | OUTPUT UNIT |

# FIG. 8

EP 3 847 961 B1

PROVISIONAL WALKING SECTION INFORMATION — 800

| PERIOD ID | START TIME | END TIME | WALKING FEATURE |
|-----------|------------|----------|-----------------|
| T'1 | 1518071413862 | 1518071421862 | ... |
| ... | ... | ... | ... |

800-1

FIG. 9

# FIG. 10

# FIG. 11

WINDOW WIDTH = 1.8 SECONDS

1102

1101

CORRELATION DEGREE = -0.05

WINDOW WIDTH = 0.6 SECONDS

1104

1103

CORRELATION DEGREE = 0.21

D

2D/N

# FIG. 12

EP 3 847 961 B1

# FIG. 13

GYRO DATA IN FRONT-BACK DIRECTION

MOTION AMOUNT: SMALL

1301

GYRO DATA IN HORIZONTAL DIRECTION

MOTION AMOUNT: LARGE

1302

EP 3 847 961 B1

# FIG. 14A

MONITORING SCREEN

PATIENT ID: P1

Gait

Sensor Data

1400

1403  1405

1404

1401

1402

# FIG. 14B

1410

MONITORING SCREEN  _ □ ☒

PATIENT ID: P1

Gait

Features

1411

1412

FIG. 15

```
                    ( START )
                        │
                        ▼
        ┌──────────────────────────────┐
        │   SELECT PROVISIONAL WALKING  │ ～S1501
        │            SECTION            │
        └──────────────────────────────┘
                        │
                        ▼
        ┌──────────────────────────────┐
        │       ACQUIRE GYRO DATA       │ ～S1502
        └──────────────────────────────┘
                        │
                        ▼
        ┌──────────────────────────────┐
        │   SPECIFY AXIAL DIRECTION IN  │ ～S1503
        │    WHICH MOTION AMOUNT IS     │
        │      LARGEST FOR LEFT FOOT    │
        └──────────────────────────────┘
                        │
                        ▼
        ┌──────────────────────────────┐
        │   SPECIFY AXIAL DIRECTION IN  │ ～S1504
        │    WHICH MOTION AMOUNT IS     │
        │      LARGEST FOR RIGHT FOOT   │
        └──────────────────────────────┘
                        │
                        ▼
        ┌──────────────────────────────┐
        │  CALCULATE FIRST CORRELATION  │ ～S1505
        │   DEGREE BETWEEN LEFT FOOT    │
        │   DATA AND RIGHT FOOT DATA    │
        └──────────────────────────────┘
                        │
                        ▼
```

S1506

FIRST CORRELATION DEGREE < FIRST THRESHOLD?  —NO→

YES ↓

S1508
SECOND CORRELATION DEGREE CALCULATION PROCESSING

S1509
SECOND CORRELATION DEGREE < SECOND THRESHOLD?

←YES

NO ↓

S1507
DETERMINE AS WALKING PERIOD

S1510
DETERMINE AS NON-WALKING PERIOD

S1511
IS THERE UNSELECTED PROVISIONAL WALKING SECTION?

YES →

NO ↓

S1512
OUTPUT DETERMINATION RESULT

( END )

# FIG. 16

```
                    ( START )
                        │
  ┌─────────────────────┼──────────────────────┐
  │   ┌─────────────────▼──────────────────┐
  │   │  SELECT AXIAL DIRECTION OF LEFT FOOT │───S1601
  │   └─────────────────┬──────────────────┘
  │ ┌───────────────────┼────────────────────┐
  │ │ ┌─────────────────▼──────────────────┐
  │ │ │ SELECT AXIAL DIRECTION OF RIGHT FOOT │───S1602
  │ │ └─────────────────┬──────────────────┘
  │ │   ┌───────────────▼──────────────────┐
  │ │   │ DIVIDE LEFT FOOT DATA INTO FIRST  │───S1603
  │ │   │            WINDOW DATA            │
  │ │   └───────────────┬──────────────────┘
  │ │   ┌───────────────▼──────────────────┐
  │ │   │ DIVIDE RIGHT FOOT DATA INTO SECOND│───S1604
  │ │   │           WINDOW DATA             │
  │ │   └───────────────┬──────────────────┘
  │ │ ┌─────────────────┼──────────────────┐
  │ │ │ ┌───────────────▼──────────────────┐
  │ │ │ │     SELECT FIRST WINDOW DATA      │───S1605
  │ │ │ └───────────────┬──────────────────┘
  │ │ │ ┌───────────────▼──────────────────┐
  │ │ │ │ CALCULATE SECOND CORRELATION DEGREE│──S1606
  │ │ │ │ BETWEEN FIRST WINDOW DATA AND SECOND│
  │ │ │ │           WINDOW DATA             │
  │ │ │ └───────────────┬──────────────────┘
  │ │ │          YES  ◇ S1607
  │ │ └───────────────◇ IS THERE UNSELECTED ◇
  │ │                  ◇ FIRST WINDOW DATA? ◇
  │ │                        │ NO
  │ │           YES   ◇ S1608
  │ └───────────────◇ IS THERE            ◇
  │                  ◇ UNSELECTED AXIAL    ◇
  │                  ◇ DIRECTION OF RIGHT FOOT? ◇
  │                        │ NO
  │             YES  ◇ S1609
  └───────────────◇ IS THERE             ◇
                   ◇ UNSELECTED AXIAL     ◇
                   ◇ DIRECTION OF LEFT FOOT? ◇
                        │ NO
            ┌───────────▼──────────────────┐
            │  SPECIFY SECOND CORRELATION  │───S1610
            │           DEGREE             │
            └───────────┬──────────────────┘
                   ( RETURN )
```

# FIG. 17

SECOND CORRELATION DEGREE

FIRST CORRELATION DEGREE

1701

1702

FIRST CORRELATION DEGREE

EP 3 847 961 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015136582 A
- JP 2007125368 A
- JP 2010172758 A
- JP 2009039466 A
- JP 2017059089 A
- US 2011246123 A1
- WO 2017199305 A